# EUROPEAN PATENT APPLICATION

(11) **EP 4 467 074 A1**
(43) Date of publication of application: **27.11.2024**
(21) Application number: 23743310.7
(22) Date of filing: 19.01.2023
(51) Int. Cl.: A61B 5/11, A61B 5/02, A61B 5/026, A61B 5/08, A61B 5/107, A61B 5/113, A61B 5/16, A61B 7/04, G16H 10/60

(54) **INFORMATION PROCESSING SYSTEM, DETECTION DEVICE, SERVER DEVICE, INFORMATION PROCESSING DEVICE, CONTROL METHOD, AND PROGRAM**

(30) Priority: 21.01.2022 JP 2022007906; 25.05.2022 JP 2022085522
(71) Applicant: SEKISUI CHEMICAL CO., LTD., Osaka-shi Osaka 530-8565 (JP)
(72) Inventor: NAKAMURA, Kouzou, Mishima-gun, Osaka 618-0021 (JP); SHIRASAKA, Yasuyuki, Mishima-gun, Osaka 618-0021 (JP); UENISHI, Akihiro, Tsukuba-shi, Ibaraki 300-4247 (JP); MATSUZAKI, Jiyunichi, Tsukuba-shi, Ibaraki 300-4247 (JP)
(74) Representative: Patentanwaltskanzlei WILHELM & BECK
(86) International application number: PCT/JP2023/001494
(87) International publication number: WO 2023/140317

(57) **Abstract**

A vibration generated by a subject is accurately measured. An information processing system (100) includes a signal extracting section (101), a determining section (102), and an output control section (103). The signal extracting section (101) extracts a characteristic signal and a body sound signal from a detection signal outputted from a sensor that detects, at a position at which the sensor is not in contact with a subject, vibrations generated by the subject. The determining section (102) determines at least one of a sleep state and a posture of the subject. The output control section (103) outputs the body sound signal that is extracted from the detection signal detected during a time period during which a state of the subject is at least one of the sleep state and a given posture.

## Description

### Technical Field

The present disclosure relates to an information processing system, a detecting device, a server device, an information processing device, and a method of controlling an information processing system, each of which is for detecting a vibration generated by a subject.

### Background Art

A variety of techniques for measuring vibrations (including breath sounds, heartbeat sounds, and the like) generated by a subject have been developed. For example, Patent Literature 2 discloses a device that (i) detects an abnormal lung sound on the basis of a sound signal which indicates sounds generated by the body of a subject and (ii) detects a disease of the subject. Further, Patent Literature 1 discloses a sensor system in which a mat having a number of sensors is disposed so as to cover a mattress.

### Citation List

### [Patent Literature]

[Patent Literature 1]
   Published Japanese Translation of PCT International Application Tokuhyo No. 2018-516616
[Patent Literature 2]
   Published Japanese Translation of PCT International Application Tokuhyo No. 2010-528725

### Summary of Invention

### Technical Problem

A vibration generated by a subject may reflect an abnormality in the body of the subject. In order to accurately diagnose the abnormality in the body of the subject, it is important to improve the accuracy of measuring the vibration generated by the subject.

In a case where, as with the device disclosed in Patent Literature 1, the vibration generated by the subject is measured with use of a sensor that is in direct contact with the skin of the subject, the subject may feel a discomfort as to the fact that the sensor is in contact with the skin. The vibration measured under a situation where the subject feels a discomfort may not indicate an appropriate value. Therefore, it may not be possible to accurately diagnose the abnormality in the body of the subject by the device disclosed in Patent Literature 1.

On the other hand, in a case where, as with the sensor system disclosed in Patent Literature 2, the vibration generated by the subject is measured with use of a sensor that is not in direct contact with the skin of the subject, detection accuracy can be lowered by turning, a sleeping posture, or the like of the subject. Moreover, the subject who lies down on a mat having a number of sensors recognizes that detection by the sensors is carried out. Therefore, there is a case where the subject intentionally controls the breath and it is not possible to measure the original vibration of the subject. Therefore, it may not be possible to accurately diagnose the abnormality in the body of the subject also by the sensor system disclosed in Patent Literature 1.

Thus, there is room for improvement in order to accurately measure the vibration generated by the subject.

### Solution to Problem

An information processing system in accordance with a first aspect of the present disclosure is an information processing system including: a signal extracting section that extracts, from a detection signal outputted from a sensor, (i) a characteristic signal which includes at least one of a heartbeat signal that indicates a heartbeat of a subject, a breathing vibration signal that indicates a breathing vibration of the subject, and a body motion signal that indicates a body motion of the subject and (ii) a body sound signal which indicates a body sound that is generated from an inside of a body of the subject, the sensor detecting, at a position at which the sensor is not in contact with the subject, vibrations generated by the subject; a sleep determining section that determines that the subject is in a sleep state, on the basis of the characteristic signal that has been extracted; and a first output control section that outputs the body sound signal during a time period during which it has been determined that the subject is in the sleep state, in a case where it has been determined that the subject is in the sleep state.

An information processing system in accordance with a second aspect of the present disclosure is an information processing system including: a signal extracting section that extracts, from a detection signal outputted from a sensor, (i) a characteristic signal which includes at least one of a heartbeat signal that indicates a heartbeat of a subject, a breathing vibration signal that indicates a breathing vibration of the subject, and a body motion signal that indicates a body motion of the subject and (ii) a body sound signal which indicates a body sound that is generated from an inside of a body of the subject, the sensor detecting, at a position at which the sensor is not in contact with the subject, vibrations generated by the subject; a posture determining section that determines that the subject is in a given posture, on the basis of at least one of the detection signal and the characteristic signal that has been extracted; and a second output control section that outputs the body sound signal during a time period during which it has been determined that the subject is in the given posture, in a case where it has been determined that the subject is in the given posture.

A detecting device in accordance with a third aspect of the present disclosure is a detecting device including: a sensor that detects, at a position at which the sensor is not in contact with a subject, vibrations generated by the subject; a signal extracting section that extracts, from a detection signal outputted from the sensor, (i) a characteristic signal which includes at least one of a heartbeat signal that indicates a heartbeat of the subject, a breathing vibration signal that indicates a breathing vibration of the subject, and a body motion signal that indicates a body motion of the subject and (ii) a body sound signal which indicates a body sound that is generated from an inside of a body of the subject; a sleep determining section that determines that the subject is in a sleep state, on the basis of the characteristic signal that has been extracted; and a first output control section that outputs the body sound signal during a time period during which it has been determined that the subject is in the sleep state, in a case where it has been determined that the subject is in the sleep state.

A detecting device in accordance with a fourth aspect of the present disclosure is a detecting device including: a sensor that detects, at a position at which the sensor is not in contact with a subject, vibrations generated by the subject; a signal extracting section that extracts, from a detection signal outputted from the sensor, (i) a characteristic signal which includes at least one of a heartbeat signal that indicates a heartbeat of the subject, a breathing vibration signal that indicates a breathing vibration of the subject, and a body motion signal that indicates a body motion of the subject and (ii) a body sound signal which indicates a body sound that is generated from an inside of a body of the subject; a posture determining section that determines that the subject is in a given posture, on the basis of at least one of the detection signal and the characteristic signal that has been extracted; and a second output control section that outputs the body sound signal during a time period during which it has been determined that the subject is in the given posture, in a case where it has been determined that the subject is in the given posture.

A server device in accordance with a fifth aspect of the present disclosure is a server device including: a health condition determining section that obtains the body sound signal which has been outputted from the detecting device in accordance with the third or fourth aspect and that determines a health condition of the subject by comparing the body sound signal and a given reference body sound signal; and an intervention encouragement outputting section that outputs an intervention encouragement notification which encourages a medical intervention with respect to the subject, in a case where the health condition determining section has determined that the health condition of the subject is an abnormal state.

An information processing device in accordance with a sixth aspect of the present disclosure is an information processing device including: a signal extracting section that obtains a detection signal outputted from a sensor which detects, at a position at which the sensor is not in contact with a subject, vibrations generated by the subject and that extracts, from the detection signal, (i) a characteristic signal which includes at least one of a heartbeat signal that indicates a heartbeat of the subject, a breathing vibration signal that indicates a breathing vibration of the subject, and a body motion signal that indicates a body motion of the subject and (ii) a body sound signal which indicates a body sound that is generated from an inside of a body of the subject; a sleep determining section that determines that the subject is in a sleep state, on the basis of the characteristic signal that has been extracted; and a first output control section that outputs the body sound signal during a time period during which it has been determined that the subject is in the sleep state, in a case where it has been determined that the subject is in the sleep state.

An information processing device in accordance with a seventh aspect of the present disclosure is an information processing device including: a signal extracting section that obtains a detection signal outputted from a sensor which detects, at a position at which the sensor is not in contact with a subject, vibrations generated by the subject and that extracts, from the detection signal, (i) a characteristic signal which includes at least one of a heartbeat signal that indicates a heartbeat of the subject, a breathing vibration signal that indicates a breathing vibration of the subject, and a body motion signal that indicates a body motion of the subject and (ii) a body sound signal which indicates a body sound that is generated from an inside of a body of the subject; a posture determining section that determines that the subject is in a given posture, on the basis of at least one of the detection signal and the characteristic signal that has been extracted; and a second output control section that outputs the body sound signal during a time period during which it has been determined that the subject is in the given posture, in a case where it has been determined that the subject is in the given posture.

A control method in accordance with an eighth aspect of the present disclosure is a control method which is carried out by one or more information processing devices, including: a signal extracting step of extracting, from a detection signal outputted from a sensor, (i) a characteristic signal which includes at least one of a heartbeat signal that indicates a heartbeat of a subject, a breathing vibration signal that indicates a breathing vibration of the subject, and a body motion signal that indicates a body motion of the subject and (ii) a body sound signal which indicates a body sound that is generated from an inside of a body of the subject, the sensor detecting, at a position at which the sensor is not in contact with the subject, vibrations generated by the subject; a sleep determining step of determining that the subject is in a sleep state, on the basis of the characteristic signal that has been extracted; and a first output controlling step of outputting the body sound signal during a time period during which it has been determined that the subject is in the sleep state, in a case where it has been determined that the subject is in the sleep state.

A control method in accordance with a ninth aspect of the present disclosure is a control method which is carried out by one or more information processing devices, including: a signal extracting step of extracting, from a detection signal outputted from a sensor, (i) a characteristic signal which includes at least one of a heartbeat signal that indicates a heartbeat of a subject, a breathing vibration signal that indicates a breathing vibration of the subject, and a body motion signal that indicates a body motion of the subject and (ii) a body sound signal which indicates a body sound that is generated from an inside of a body of the subject, the sensor detecting, at a position at which the sensor is not in contact with the subject, vibrations generated by the subject; a posture determining step of determining that the subject is in a given posture, on the basis of at least one of the detection signal and the characteristic signal that has been extracted; and a second output controlling step of outputting the body sound signal during a time period during which it has been determined that the subject is in the given posture, in a case where it has been determined that the subject is in the given posture.

A control method in accordance with a tenth aspect of the present disclosure is a control method which is carried out by one or more server devices, including: a health condition determining step of obtaining the body sound signal which has been outputted from the one or more information processing devices each of which carries out the control method in accordance with the eighth or ninth aspect and determining a health condition of the subject by comparing the body sound signal and a given reference body sound signal; and an intervention encouragement outputting step of outputting an intervention encouragement notification which encourages a medical intervention with respect to the subject, in a case where it has been determined, in the health condition determining step, that the health condition of the subject is an abnormal state.

The information processing systems, the detecting devices, the server device, and the information processing devices in accordance with the aspects of the present disclosure may be each realized by a computer. In this case, the present disclosure also encompasses, in its scope, (i) a control program for the information processing systems, the detecting devices, the server device, and the information processing devices, the control program causing the computer to realize the information processing systems, the detecting devices, and the server device by causing the computer to operate as each section (software element) of the information processing systems, the detecting devices, the server device, and the information processing devices and (ii) a computer-readable recording medium in which the control program is recorded.

### Advantageous Effects of Invention

According to an aspect of the present disclosure, it is possible to accurately measure a vibration generated by a subject.

### Brief Description of Drawings

Fig. 1 is a conceptual diagram illustrating an example of a configuration of an information processing system.
Fig. 2 is a view illustrating an example of a schematic configuration of a detecting device.
Fig. 3 is a view illustrating a different example of the schematic configuration of the detecting device.
Fig. 4 is a view illustrating another example of the schematic configuration of the detecting device.
Fig. 5 is a functional block diagram illustrating an example of a configuration of an information processing system.
Fig. 6 is a view explaining various signals that are included in a detection signal.
Fig. 7 is a view explaining (i) various sounds that can be included in a lung sound and (ii) typical cases that correspond to the various sounds.
Fig. 8 is a flowchart illustrating an example of a flow of a process carried out by the information processing system.
Fig. 9 is a flowchart illustrating a different example of the flow of the process carried out by the information processing system.
Fig. 10 is a functional block diagram illustrating an example of a configuration of an information processing system.
Fig. 11 is a flowchart illustrating an example of a flow of a process carried out by the information processing system.
Fig. 12 is a functional block diagram illustrating an example of a configuration of an information processing system.
Fig. 13 is a flowchart illustrating an example of a flow of a process carried out by the information processing system.
Fig. 14 is a flowchart illustrating an example of a flow of a process of determining a health condition of a subject which process is carried out by the information processing system.
Fig. 15 is a view illustrating an example of a display screen displayed on a display section of a communication device.
Fig. 16 is a functional block diagram illustrating an example of a configuration of an information processing system.
Fig. 17 is a functional block diagram illustrating an example of a configuration of an information processing system.
Fig. 18 is a functional block diagram illustrating an example of a configuration of an information processing system.
Fig. 19 is a schematic view for explaining a model of a neuron.
Fig. 20 is a view for explaining a neural network.
Fig. 21 is a sonogram showing a body sound of a healthy person during awaking (immediately after the healthy person went to bed).
Fig. 22 is a sonogram showing an example of a body sound signal of the healthy person during sleeping.
Fig. 23 is a sonogram showing an example of a body sound signal of a healthy person (left lateral decubitus position).
Fig. 24 is a sonogram showing an example of a body sound signal of the healthy person (supine position).
Fig. 25 is a sonogram showing an example of a lung sound signal of a healthy person which lung sound signal included a fine crackle signal.
Fig. 26 is a sonogram showing an example of a lung sound signal of a non-healthy person which lung sound signal included a coarse crackle signal.
Fig. 27 is a sonogram obtained by extracting only a high-strength portion of a lung sound signal of a healthy person (supine position).
Fig. 28 is a sonogram obtained by extracting only a high-strength portion of a lung sound signal of a non-healthy person which lung sound signal included a fine crackle signal.
Fig. 29 is a sonogram obtained by extracting only a high-strength portion of a lung sound signal of a non-healthy person which lung sound signal included a coarse crackle signal.
Fig. 30 is a view showing an example of a power spectrum of a lung sound signal of a healthy person (supine position).
Fig. 31 is a view showing an example of a power spectrum of a lung sound signal of a non-healthy person which lung sound signal included a fine crackle signal.
Fig. 32 is a view showing an example of a power spectrum of a lung sound signal of a non-healthy person which lung sound signal included a coarse crackle signal.
Fig. 33 is a sonogram showing an example of a heartbeat signal of a healthy person.
Fig. 34 is a sonogram showing an example of a body motion signal of the healthy person.
Fig. 35 is a sonogram showing an example of a breath sound signal of the healthy person (during awaking).
Fig. 36 is a sonogram showing an example of a breath sound signal of the healthy person (during sleeping).

### Description of Embodiments

### Embodiment 1

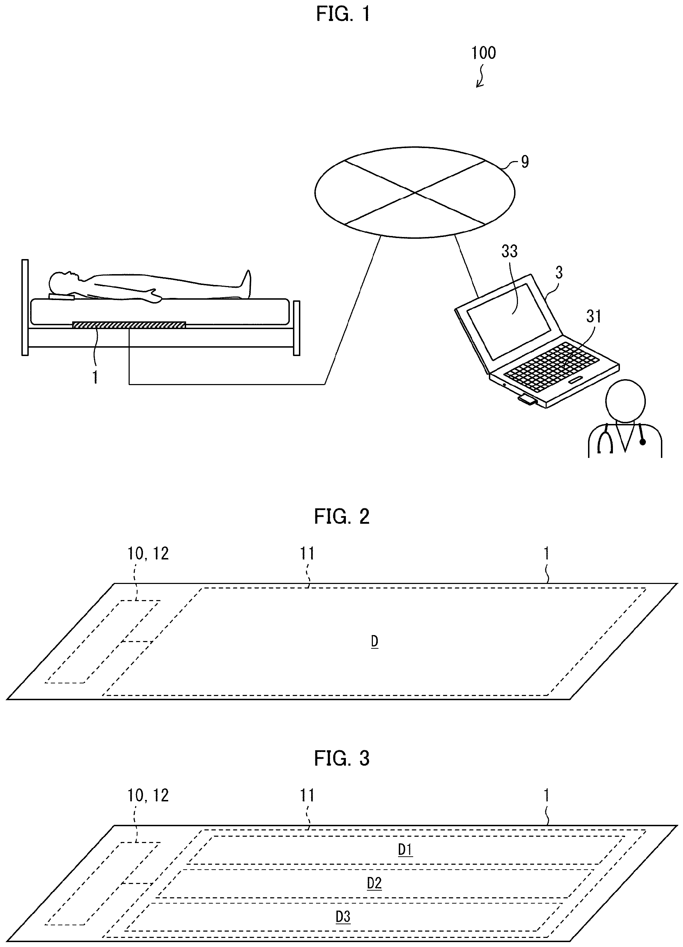

The following description will discuss an embodiment of the present disclosure in detail with reference to Figs. 1 to 9.

### (Outline of information processing system 100)

An information processing system 100 in accordance with Embodiment 1 of the present disclosure is a system that outputs at least one of (i) a body sound signal of a subject while the subject is in a sleep state and (ii) a body sound signal of the subject while the subject is in a given posture, on the basis of a detection signal outputted from a sensor that detects, at a position at which the sensor is not in contact with the subject, vibrations generated by the subject.

In this specification, the "subject" is typically a person who needs to be monitored by a medical expert and others, such as a patient who lies down on a bed or the like. The "detection signal" is a signal that indicates the vibrations generated by the subject, and is raw data outputted from the sensor or data obtained by subjecting the raw data to an amplification process or a noise removal process. The noise removal process can be carried out, for example, by carrying out a filtering process with respect to a region of not less than 2,000 Hz of the raw data. The "given posture" is a good posture that is suitable for detection carried out by the sensor, and is, for example, at least one of a right lateral decubitus position, a left lateral decubitus position, a supine position, a prone position, and a sitting position. The "body sound signal" is a signal that indicates a body sound generated from the inside of the body of the subject, and is a signal including at least one of a lung sound signal that indicates a lung sound of the subject, a blood flow sound signal that indicates a blood flow sound of the subject, a heart sound signal that indicates a sound of the heart of the subject, a bowel sound signal that indicates a bowel sound of the subject, and a peristaltic sound signal that indicates a peristaltic sound of the subject.

### (Configuration of information processing system 100)

First, a schematic configuration of the information processing system 100 in accordance with Embodiment 1 of the present disclosure is described with reference to Fig. 1. Fig. 1 is a conceptual diagram illustrating an example of the configuration of the information processing system 100.

As illustrated in Fig. 1, the information processing system 100 may include a detecting device 1 (information processing device) and a communication device 3 (information processing device). The information processing system 100 may include a single detecting device 1 or may alternatively include a plurality of detecting devices 1. Similarly, the information processing system 100 may include a single communication device 3 or may alternatively include a plurality of communication devices 3.

The detecting device 1 analyzes the detection signal outputted from a sensor 11 (Fig. 2 etc.) that detects, at a position at which the sensor 11 is not in contact with the subject, the vibrations generated by the subject and that indicates the vibrations generated by the subject, and determines that a state of the subject is at least one of (a) the sleep state and (b) the given posture.

Then, the detecting device 1 outputs, to the outside, the body sound signal that has been extracted from the detection signal detected during a time period during which the above determination is made. A destination to which the body sound signal is outputted is typically the communication device 3. The communication device 3 is typically a computer, a smartphone, a tablet terminal, or the like that is used by, for example, the medical expert and others, and is set in, for example, a nurses station.

Note that the detecting device 1 extracts the body sound signal and the like from the detection signal by carrying out a process, such as a frequency analysis, with respect to the detection signal outputted from the sensor 11. This extraction process is carried out independently of a process of determining the above (a) or (b), and is typically carried out prior to this determination process.

The sensor 11 is set at the position at which the sensor 11 is not in contact with the subject so that the subject does not feel a discomfort. The sensor 11 may be set on the bed on which the subject lies down. The position at which the sensor 11 is set may be between the bed on which the subject lies down and a mattress on the bed or between the mattress and a bed sheet on the mattress. In a case where the subject wears clothes, the position at which the sensor 11 is set may be the uppermost surface of the bed. In a case where the sensor 11 is set at any of these positions, the sensor 11 is preferably formed in a thin plate (sheet)-like shape.

As has been described, since, in the information processing system 100, the sensor 11 that is set at the position at which the sensor 11 is not in contact with the subject is used, the subject does not feel a discomfort. Therefore, it is possible to suppress a factor in decreasing detection accuracy, such as the subject's intentionally controlling breathing, and also possible to realize constant monitoring. Moreover, since, under such circumstances, the body sound signal while the subject is in the sleep state or the body sound signal while the subject is in the given posture is outputted in the information processing system 100, it is possible to output the body sound signal that is more accurate than a conventional one. As a result, it is possible to accurately diagnose an abnormality in the body of the subject.

Note that the detecting device 1 and the communication device 3 may be directly connected to each other or may be alternatively communicably connected to each other with a communication network 9 therebetween as illustrated in Fig. 1 The communication network 9 is not limited to any form, and may be a local area network (LAN) or the Internet.

Note also that the information processing system 100 may include a server device (information processing device) (not illustrated) that is communicably connected to the detecting device 1 and the communication device 3, in addition to the detecting device 1 and the communication device 3. For example, the server device may be configured to store and manage, for each subject, a body sound signal transmitted from each of a plurality of detecting devices 1. The medical expert and others may be able to access the server device with use of the communication device 3 and refer to the body sound signal of each subject.

### (Configuration of detecting device 1)

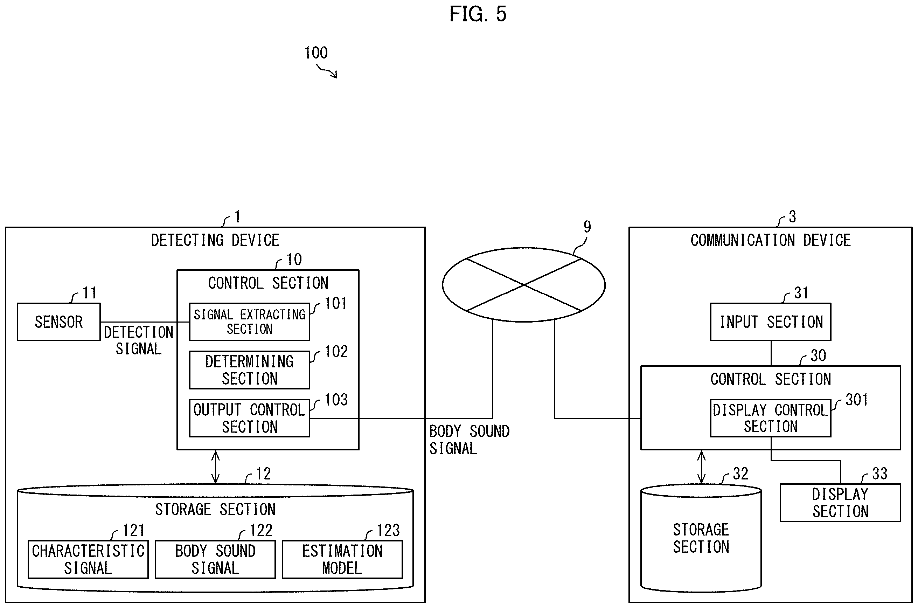

Next, an appearance and a schematic configuration of the detecting device 1 are described with use of Fig. 5 with reference to Figs. 2 to 4 and Fig. 6. Fig. 5 is a functional block diagram illustrating an example of the configuration of the information processing system 100. A case where a characteristic signal includes a rhonchi signal is described here as an example. However, the characteristic signal is not limited to this example, and may not include the rhonchus signal.

As illustrated in Fig. 5, the detecting device 1 includes the sensor 11, a control section 10, and a storage section 12.

### [Sensor 11]

The sensor 11 is a noncontact (noninvasive) sensor that is capable of detecting, at the position at which the sensor 11 is not in contact with the subject, the vibrations generated by the subject. The sensor 11 outputs, for example, the detection signal that indicates the vibrations (i.e., waveform data). The noncontact (noninvasive) sensor is not limited to any particular type. For example, as the noncontact (noninvasive) sensor, a piezoelectric sensor, a Doppler sensor, or the like can be applied.

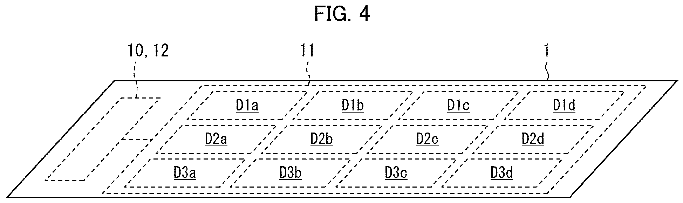

The sensor 11 may include one or more detection regions. In a case where the sensor 11 includes a plurality of detection regions, the sensor 11 may output the detection signal that has been detected in each of the plurality of detection regions. In a case where the sensor 11 is formed in a thin plate-like shape, the plurality of detection regions may be arranged side by side on an identical plane. Figs. 2 to 4 are each a view illustrating an example of the schematic configuration of the detecting device 1. The detecting device 1 illustrated in Fig. 2 includes the sensor 11 that includes a single detection region D. The detecting device 1 illustrated in Fig. 3 includes the sensor 11 that includes detection regions D1 to D3 arranged in three columns. The detecting device 1 illustrated in Fig. 4 includes the sensor 11 that includes detection regions D1a to D4d arranged in four rows and three columns. For example, in the case of the detecting device 1 illustrated in Fig. 3, the detection signal that has been detected in each of the detection regions D1 to D4 is outputted individually. Similarly, in the case of the detecting device 1 illustrated in Fig. 4, the detection signal that has been detected in each of the detection regions D1a to D3d is outputted individually. Each of the detection regions D1a to D3d may be, for example, a 10 cm square.

In a case where the configuration in which the sensor 11 includes the plurality of detection regions is employed, it is possible to accurately specify a position in the body of the subject at which position an abnormality occurs, by individually analyzing the detection signal from each of the plurality of detection regions and comparing results of analyses with each other. Moreover, it is possible to accurately specify the posture of the subject on the basis of the signal strength or the like of the detection signal from each of the detection regions.

### [Control section 10 and storage section 12]

The control section 10 may be a central processing unit (CPU) in an example. The control section 10 carries out various functions by reading a control program that is software stored in the storage section 12 and loading the control program in a memory such as a random access memory (RAM). As illustrated in Fig. 5, the control section 10 includes a signal extracting section 101, a determining section 102 (sleep determining section, posture determining section), and an output control section 103 (first output control section, second output control section). Note that, in the storage section 12 illustrated in Fig. 5, the control program is not illustrated for simplification of description.

The signal extracting section 101 extracts, from the detection signal outputted from the sensor 11, the characteristic signal of the subject and the body sound signal that indicates the body sound generated from the inside of the body of the subject. Note, here, that the characteristic signal includes at least one of a heartbeat signal that indicates a heartbeat of the subject, a breathing vibration signal that indicates a breathing vibration of the subject, and a body motion signal that indicates a body motion of the subject. The characteristic signal may further include the rhonchus signal that indicates a rhonchus of the subject. Note, here, that the rhonchus is a kind of adventitious sound included in the lung sound, and is a snore-like sound generated from a lung. The signal extracting section 101 extracts the characteristic signal and the body sound signal from the detection signal.

The sensor 11 can detect the vibrations in various frequency ranges which vibrations occur from the subject. Therefore, the detection signal outputted from the sensor 11 is a signal in which a plurality of vibrations having various frequency characteristics overlap with each other. In an example, the sensor 11 may be capable of obtaining at least one of the body sound signal that has a frequency of not less than 100 Hz, the heartbeat signal that has a frequency of not more than 20 Hz, the breathing vibration signal, and the body motion signal. The sensor 11 may be capable of obtaining the rhonchus signal that has a frequency of not less than 100 Hz. Note that it is possible to differentiate the body sound signal that has a frequency of not less than 100 Hz and the rhonchus signal that has a frequency of not less than 100 Hz on the basis of frequency components (spectra).

In this manner, the detecting device 1 may include the sensor 11 that can detect the vibrations in various frequency bands. This makes it unnecessary to provide a plurality of differing types of sensors to the detecting device 1, and makes it easy for the medical expert and others who use the information processing system 100 to maintain and manage the detecting device 1. Therefore, the convenience of the detecting device 1 is improved.

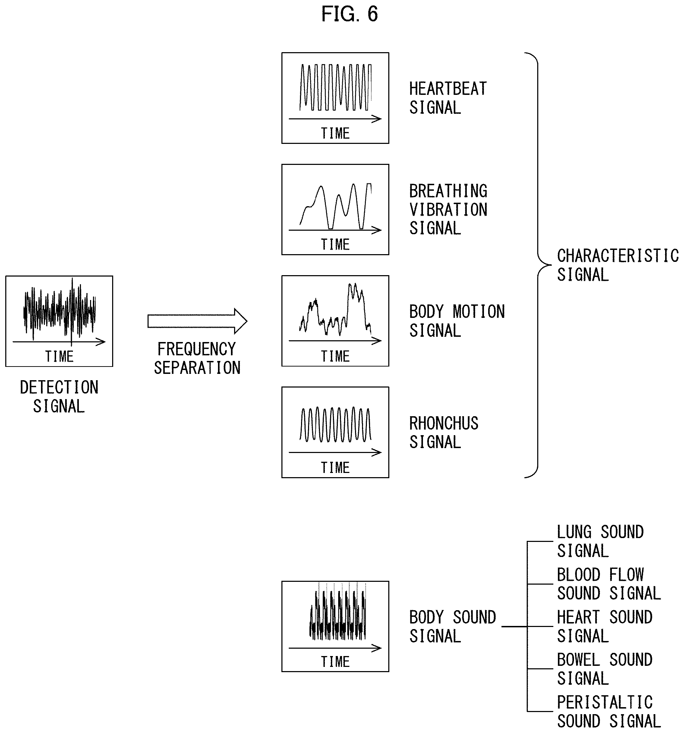

The signal extracting section 101 may extract the characteristic signal and the body sound signal from the detection signal by applying a well-known technique, such as frequency separation, to the detection signal. This is described with reference to Fig. 6. Fig. 6 is a view explaining various signals that are included in the detection signal. As illustrated in Fig. 6, by carrying out frequency separation, it is possible to separate the detection signal into the heartbeat signal, the breathing vibration signal, the body motion signal, the rhonchus signal, and the body sound signal which differ from each other in frequency characteristic.

The body sound signal includes at least one of the lung sound signal that indicates the lung sound of the subject, the blood flow sound signal that indicates the blood flow sound of the subject, the heart sound signal that indicates the sound of the heart of the subject, the bowel sound signal that indicates the bowel sound of the subject, and the peristaltic sound signal that indicates the peristaltic sound of the subject. The lung sound signal, the blood flow sound signal, the heart sound signal, the bowel sound signal, and the peristaltic sound signal differ from each other in frequency characteristic.

The signal extracting section 101 stores, in the storage section 12, the characteristic signal and the body sound signal that have been extracted from the detection signal (121 and 122 illustrated in Fig. 5). The characteristic signal and the body sound signal may be stored together with time information that indicates a time at which the detection signal from which the characteristic signal and the body sound signal are extracted has been detected.

The determining section 102 determines at least one of the following: the subject is in the sleep state; and the subject is in the given posture. In a case where the determining section 102 determines that the subject is in the sleep state, the determining section 102 uses the characteristic signal that has been extracted. In a case where the determining section 102 determines that the subject is in the given posture, the determining section 102 uses at least one of the detection signal and the characteristic signal that has been extracted. That is, the determining section 102 may use any one of the detection signal, the heartbeat signal, the breathing vibration signal, and the body motion signal so as to determine the given posture of the subject. For example, in a case where the characteristic signal further includes the rhonchus signal, the determining section 102 may use any one of the detection signal, the heartbeat signal, the breathing vibration signal, the body motion signal, and the rhonchus signal so as to determine the given posture of the subject. Note that a rate of contribution of each of the detection signal, the heartbeat signal, the breathing vibration signal, the body motion signal, and the rhonchus signal to the determination of the given posture of the subject may not be the same. Note that the determining section 102 may determine that the subject is in the sleep state and the given posture.

In a case where the determining section 102 determines the sleep state of the subject, the determining section 102 may estimate the sleep state by inputting, into an estimation model 123, the characteristic signal that has been extracted by the signal extracting section 101. The estimation model 123 has been trained by machine learning with use of training data in which the characteristic signal is an explanatory variable and the sleep state is a response variable. In a case where the determining section 102 determines the posture of the subject, the determining section 102 may estimate a probability that the subject is in the given posture by inputting, into the estimation model 123, the detection signal that has been detected by the sensor 11. The estimation model 123 has been trained by machine learning with use of training data in which the detection signal is an explanatory variable and the posture of the subject is a response variable. Alternatively, in a case where the determining section 102 determines the posture of the subject, the determining section 102 may estimate the probability that the subject is in the given posture by inputting, into the estimation model 123, the characteristic signal that has been extracted by the signal extracting section 101. The estimation model 123 has been trained by machine learning with use of training data in which the characteristic signal is an explanatory variable and the posture of the subject is a response variable. In these cases, the determining section 102 determines that the subject is in the given posture, on the basis of the probability obtained as an estimation result. To the machine learning for generating the estimation model 123, a publicly known machine learning algorithm, such as a neural network or a support vector machine, can be applied.

Note that a place at which the estimation model 123 is present is not limited, and may be stored in the storage section 12 as illustrated in Fig. 5 or may be alternatively stored in a device other than the detecting device 1.

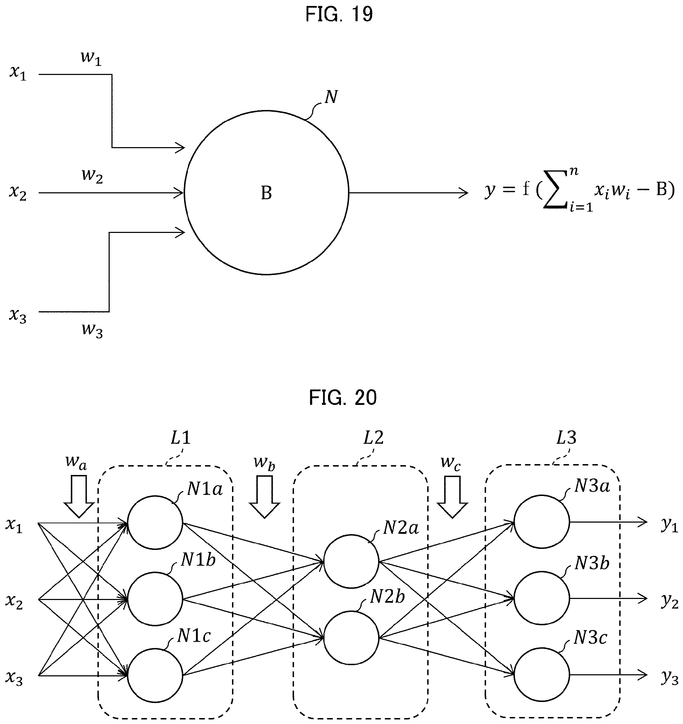

A case where a neural network is used for the estimation model 123 is described here with reference to Figs. 19 and 20. The neural network is constituted, for example, by a processor, a memory, and the like that realize a neural network which mimics a model of a neuron illustrated in Fig. 19. Fig. 19 is a schematic view for explaining the model of the neuron. Fig. 20 is a view for explaining the neural network.

The neural network is constituted by, for example, an input layer that includes a plurality of neurons, a hidden layer (intermediate layer) that includes a plurality of neurons, and an output layer that includes a plurality of neurons. Each neuron outputs a result y with respect to a plurality of inputs x, as illustrated in Fig. 19. Each of the inputs x is multiplied by a corresponding weighting factor w. For example, in Fig. 19, an input x₁ is multiplied by a weighting factor w₁, an input x₂ is multiplied by a weighting factor w₂, and an input x₃ is multiplied by a weighting factor w₃. The neuron sums up results obtained by multiplying each of the inputs by the weighting factor, and outputs the result y by substituting, into an activation function f, a result obtained by considering a bias B with respect to the summed result.

Next, the neural network obtained by combining these neurons is described with reference to Fig. 20. Fig. 20 is a schematic view illustrating a neural network that has an input layer L1, a hidden layer L2, and an output layer L3. In the neural network illustrated in Fig. 20, a plurality of inputs x are inputted into the input layer L1, and results y are outputted from the output layer L3. The neural network may has a plurality of hidden layers. In Fig. 20, for example, each of inputs x₁ to x₃ is multiplied by a corresponding weighting factor wₐ. Results obtained by such multiplication are each inputted into each of three neurons N1a to N1c. Note, here, that the neurons N1a to N1c respectively output p₁₁ to p₁₃. Vectors (p₁₁, p₁₂, and p₁₃) can be respectively regarded as feature vectors obtained by extracting features of input vectors (x₁, x₂, and x₃). These feature vectors (p₁₁, p₁₂, and p₁₃) are feature vectors between the input layer L1 and the hidden layer L2.

Each of p₁₁ to p₁₃ is multiplied by a corresponding weighting factor w_{b}. Results obtained by such multiplication are each inputted into each of two neurons N2a and Nb. Note, here, that the neurons N2a and N2b respectively output p₂₁ and p₂₂. These vectors (p₂₁ and p₂₂) are feature vectors between the hidden layer L2 and the output layer L3.

Each of p₂₁ and p₂₂ is multiplied by a corresponding weighting factor w_{c}. Results obtained by such multiplication are each inputted into each of three neurons N3a to N3c. The neurons N3a to N3c respectively output results y₁ to y₃.

Operations of the neural network include an operation in a training mode and an operation in an estimation mode. In the training mode, the neural network trains (adjusts) a parameter that indicates the weighting factor w with use of training data including an explanatory variable and a response variable. In the estimation mode, the neural network outputs a result estimated from inputted data (e.g., characteristic signal) with use of the parameter adjusted by training.

In the training mode, an error between (i) a result outputted from the output layer L3 in a case where the explanatory variable included in the training data is inputted into the input layer L1 and (ii) the response variable corresponding to the explanatory variable is calculated, and the parameter is adjusted so that this error is reduced.

To adjustment of the parameter, any known method can be applied. For example, a backpropagation method may be applied. Until the error falls within a given range or until all explanatory variables included in the training data are inputted, the adjustment of the parameter may be repeated.

Returning to Fig. 5, the output control section 103 outputs the body sound signal that has been extracted from the detection signal detected during at least one of a time period during which it has been determined that the subject is in the sleep state and a time period during which it has been determined that the subject is in the given posture. For example, in a case where it has been determined that the subject is in the sleep state, the output control section 103 may output only the body sound signal that has been extracted from the detection signal detected during the time period during which it has been determined that the subject is in the sleep state, among body sound signals stored in the storage section 12. For example, in a case where it has been determined that the subject is in the given posture, the output control section 103 may output only the body sound signal that has been extracted from the detection signal detected during the time period during which it has been determined that the subject is in the given posture, among the body sound signals stored in the storage section 12. For example, in a case where it has been determined that the subject is in the sleep state and the given posture, the output control section 103 may output only the body sound signal that has been extracted from the detection signal detected during a time period during which it has been determined that the subject is in the sleep state and it has been determined that the subject is in the given posture, among the body sound signals 122 stored in the storage section 12.

### (Configuration of communication device 3)

Next, a configuration of the communication device 3 is described with reference to Fig. 5. The communication device 3 includes an input section 31, a control section 30, a storage section 32, and a display section 33.

The input section 31 may be a keyboard, a touch panel, a mouse, or the like. The control section 30 carries out various functions by reading a control program that is software stored in the storage section 32 and loading the control program in a memory such as a RAM. The control section 30 includes a display control section 301 that causes various pieces of information to be displayed on the display section 33.

### (Modification in configuration of information processing system 100)

The signal extracting section 101 may be capable of further extracting the lung sound signal, the blood flow sound signal, the heart sound signal, the bowel sound signal, the peristaltic sound signal, and the like from the body sound signal that has been extracted from the detection signal detected during at least one of the time period during which it has been determined that the subject is in the sleep state and the time period during which it has been determined that the subject is in the given posture. In this case, the detecting device 1 may output, together with the body sound signal, the lung sound signal, the blood flow sound signal, the heart sound signal, the bowel sound signal, the peristaltic sound signal, and the like that have been extracted.

The communication device 3 may have a function of extracting, from the body sound signal that has been outputted by the detecting device 1, the lung sound signal, the blood flow sound signal, the heart sound signal, the bowel sound signal, the peristaltic sound signal, and the like (for example, a function similar to that of the signal extracting section 101 of the detecting device 1). Alternatively, the detecting device 1 may extract, from the body sound signal for output, the lung sound signal, the blood flow sound signal, the heart sound signal, the bowel sound signal, and the peristaltic sound signal, and output, together with the body sound signal, the lung sound signal, the blood flow sound signal, the heart sound signal, the bowel sound signal, and the peristaltic sound signal that have been extracted. In a case where the information processing system 100 includes the server device in addition to the detecting device 1 and the communication device 3, the server device may have a function of extracting, from the body sound signal that has been outputted from the detecting device 1, the lung sound signal, the blood flow sound signal, the heart sound signal, the bowel sound signal, the peristaltic sound signal, and the like.

Whichever of these configurations is employed, it is possible to extract a signal important for diagnosing the health condition of the subject, on the basis of the body sound signal that has been accurately measured by the detecting device 1.

The detecting device 1 may be configured to output the detection signal detected during at least one of the time period during which it has been determined that the subject is in the sleep state and the time period during which it has been determined that the subject is in the given posture. In this case, for example, the communication device 3 or the server device only needs to have a function of extracting the body sound signal from the detection signal obtained from the detecting device 1 (for example, a function similar to that of the signal extracting section 101 of the detecting device 1).

### (Correspondence between abnormalities in body sound signal and diseases)

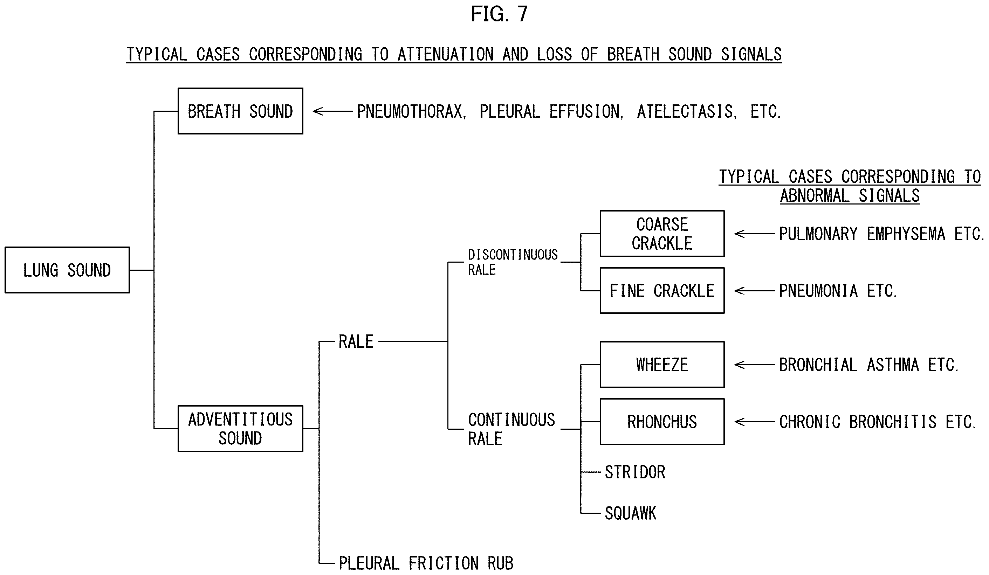

A reason why accurate measurement of the body sound signal of the subject is important for accurate diagnosis of the health condition of the subject is described with reference to a specific example. Fig. 7 is a view explaining (i) various sounds that can be included in the lung sound and (ii) typical cases that correspond to the various sounds.

The lung sound is a sound included in the body sound. The lung sound signal can be extracted from the body sound signal by subjecting the body sound signal to a frequency analysis. The lung sound includes a breath sound and an adventitious sound.

In a case where the breath sound of the subject is attenuated and lost as compared with that of a healthy person, it is possible that the subject is affected by a disease such as air bubbles, pleural effusion, and atelectasis. In a case where an exhalation segment of the subject is prolonged and in a case where an abnormal sound is mixed in the breath sound, it is possible that the subject is affected by chronic obstructive pulmonary disease (COPD) and bronchial asthma.

On the other hand, the adventitious sound includes a "rale", "pleural friction rub", and the like. The "rale" includes a discontinuous rale and a continuous rale. The discontinuous rale includes a coarse crackle and a fine crackle. The continuous rales include a wheeze, a rhonchus, a stridor, and a squawk. That is, the adventitious sound included in the body sound of the subject includes at least one of the coarse crackle, the fine crackle, the wheeze, the rhonchus, the stridor, the squawk, and the pleural friction rub of the subject.

In a case where the coarse crackle is mixed in the lung sound of the subject, it is possible that the subject is affected by pulmonary emphysema or the like. In a case where the fine crackle is mixed in the lung sound of the subject, it is possible that the subject is affected by pneumonia or the like. In a case where the wheeze is mixed in the lung sound of the subject, it is possible that the subject is affected by bronchial asthma or the like. In a case where the rhonchus is mixed in the lung sound of the subject, it is possible that the subject is affected by chronic bronchitis or the like.

In this manner, it is possible to estimate and detect, at an early stage, a disease by which the subject may be affected, on the basis of the lung sound of the subject. Fig. 7 explains the lung sound, as an example. It is also possible to estimate and diagnose the health condition of the subject on the basis of the blood flow sound, the heart sound, the bowel sound, and the peristaltic sound. Note, however, that, in order to accurately diagnose the health condition of the subject, it is necessary to accurately measure the body sound signal. By employing the information processing system 100 in accordance with the present disclosure, it is possible to accurately measure the body sound signal of the subject, and it is possible to set the body sound signal as an analysis target. Therefore, it is possible for the medical expert and others to highly accurately diagnose the health condition of the subject.

### (Process carried out by information processing system 100)

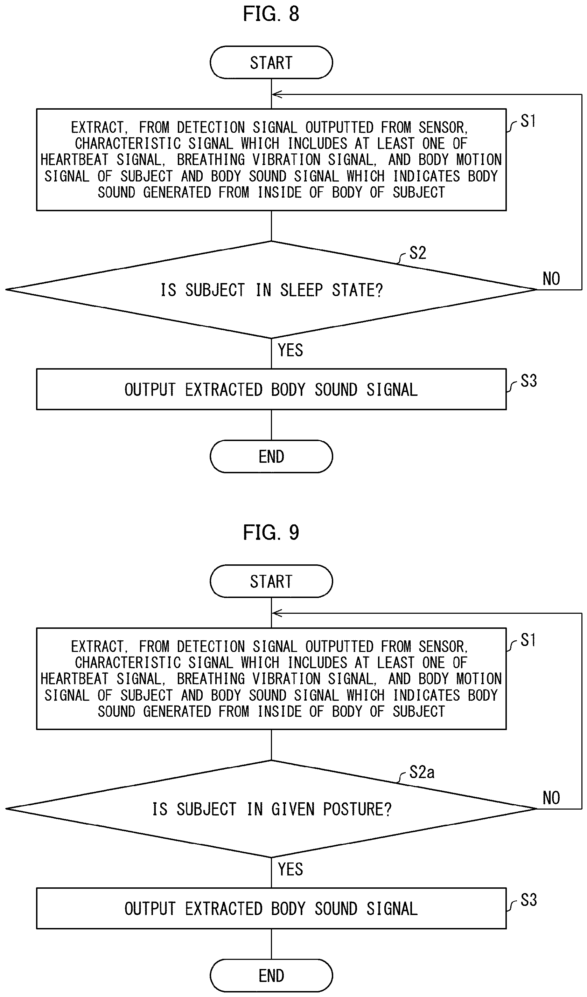

Next, a flow of a process carried out by the information processing system 100 is described with reference to Figs. 8 and 9. Figs. 8 and 9 are each a flowchart illustrating an example of the flow of the process carried out by the information processing system 100 (e.g., the detecting device 1).

Fig. 8 illustrates an example process carried out by the information processing system 100 in a case where the body sound signal that has been extracted from the detection signal detected during the time period during which it has been determined that the subject is in the sleep state is outputted. First, the signal extracting section 101 extracts the characteristic signal and the body sound signal from the detection signal outputted from the sensor 11 (step S1: signal extracting step).

Next, the determining section 102 (sleep determining section) determines that the subject is in the sleep state, on the basis of the characteristic signal that has been extracted (step S2: sleep determining step). In a case where it has been determined that the subject is not in the sleep state (NO in the step S2), the process returns to the step S1.

In a case where it has been determined that the subject is in the sleep state (YES in the step S2), the output control section 103 (first output control section) outputs the body sound signal that has been extracted from the detection signal detected during the time period during which it has been determined that the subject is in the sleep state (step S3: first output controlling step).

Fig. 9 illustrates an example process carried out by the information processing system 100 in a case where the body sound signal that has been extracted from the detection signal detected during the time period during which it has been determined that the subject is in the given posture is outputted. First, the signal extracting section 101 extracts the characteristic signal and the body sound signal from the detection signal outputted from the sensor 11 (step S1: signal extracting step).

Next, the determining section 102 (posture determining section) determines that the subject is in the given posture, on the basis of at least one of the detection signal and the characteristic signal that has been extracted (step S2a: posture determining step). In a case where it has been determined that the subject is not in the given posture (NO in the step S2a), the process returns to the step S1.

In a case where it has been determined that the subject is in the given posture (YES in the step S2a), the output control section 103 (second output control section) outputs the body sound signal that has been extracted from the detection signal detected during the time period during which it has been determined that the subject is in the given posture (step S3: second output controlling step).

Note that a timing at which the information processing system 100 carries out the process illustrated in each of Figs. 8 and 9 can be set as desired. For example, the information processing system 100 may carry out the process illustrated in each of Figs. 8 and 9 at given intervals (e.g., every hour) or every time the sensor 11 determines that the subject leaves the bed.

### Embodiment 2

The following description will discuss another embodiment of the present disclosure. Note that, for convenience, members having the same functions as those of the members described in the above embodiment will be given the same reference signs and descriptions thereof will not be repeated.

### (Configuration of information processing system 100a)

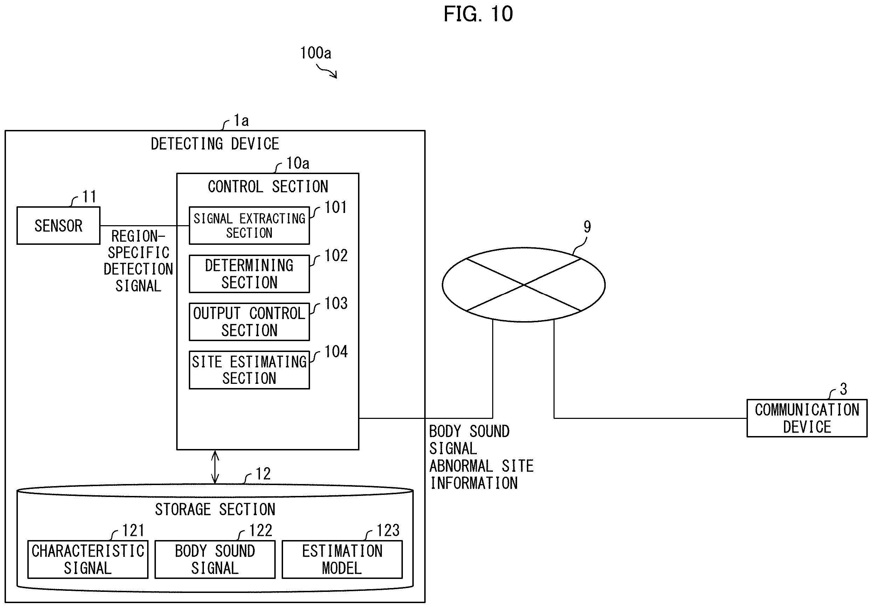

With reference to Fig. 10, described is an information processing system 100a that employs a configuration in which a sensor 11 includes a plurality of detection regions and outputs detection signals that have been detected in the respective plurality of detection regions (hereinafter, region-specific detection signals). Fig. 10 is a functional block diagram illustrating an example of the configuration of the information processing system 100a.

As illustrated in Fig. 10, the information processing system 100a may include a detecting device 1a (information processing device) and a communication device 3 (information processing device). The detecting device 1a includes the sensor 11 that includes the plurality of detection regions, a control section 10a, and a storage section 12. The control section 10a includes a site estimating section 104, in addition to a signal extracting section 101, a determining section 102 (sleep determining section, posture determining section), and an output control section 103 (first output control section, second output control section).

The site estimating section 104 estimates an abnormal sound occurring site in the body of a subject, on the basis of a body sound signal that has been extracted from each of the region-specific detection signals.

Here, the configuration in which the detecting device 1a includes the site estimating section 104 has been described as an example. However, the information processing system 100a is not limited to this example. For example, the communication device 3 or a server device (information processing device) that is not illustrated may have a function similar to that of the site estimating section 104.

### (Process carried out by information processing system 100a)

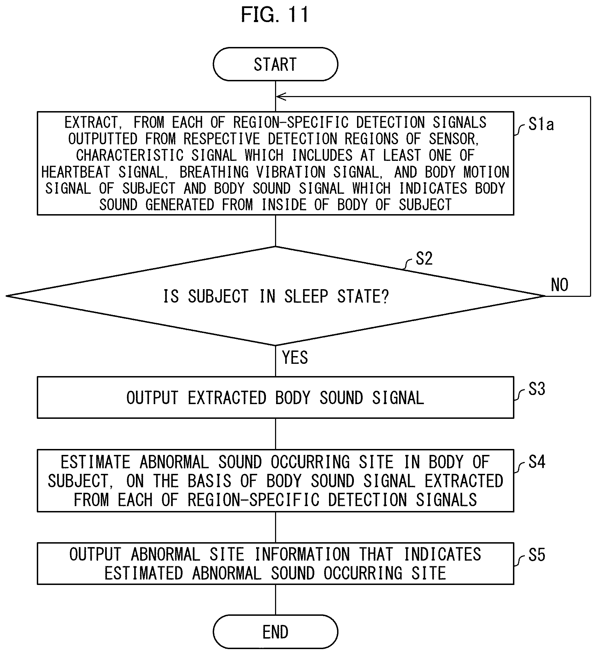

Next, a flow of a process carried out by the information processing system 100a is described with reference to Fig. 11. Fig. 11 is a flowchart illustrating an example of the flow of the process carried out by the information processing system 100a. A case where the determining section 102 of the information processing system 100a determines that the subject is in a sleep state is described here as an example. However, the information processing system 100a is not limited to this example. The determining section 102 of the information processing system 100a may be configured to determine that the subject is in a given posture.

The signal extracting section 101 obtains the region-specific detection signals outputted from the respective detection regions (e.g., detection regions D1 to D4 illustrated in Fig. 3), and extracts a characteristic signal and the body sound signal from each of the region-specific detection signals (step S1a: signal extracting step).

Next, the determining section 102 determines that the subject is in the sleep state, on the basis of the characteristic signal that has been extracted from each of the region-specific detection signals (step S2: sleep determining step). In a case where it has been determined that the subject is not in the sleep state (NO in the step S2), the process returns to the step S1.

In a case where it has been determined that the subject is in the sleep state (YES in the step S2), the output control section 103 (first output control section) outputs the body sound signal that has been extracted from each of the region-specific detection signals detected during a time period during which it has been determined that the subject is in the sleep state (step S3: first output controlling step).

The site estimating section 104 estimates the abnormal sound occurring site in the body of the subject, on the basis of the body sound signal that has been extracted from each of the region-specific detection signals (step S4). For example, the site estimating section 104 may specify the position of the detection region which has outputted the region-specific detection signal from which the body sound signal that includes the largest number of abnormal sounds has been extracted, and estimate, as the abnormal sound occurring site, a site in the body of the subject which site is estimated to be close to the specified position.

Next, the output control section 103 outputs abnormal site information that indicates the estimated abnormal sound occurring site (step S5).

In a case where this configuration is employed, it is possible for the information processing system 100a to accurately measure the body sound signal of the subject and specify the abnormal sound occurring site associated with a disease of the subject. For example, in a case where the outputted body sound signal and the outputted abnormal site information are provided to a medical expert and others, it is possible for the medical expert and others to high accurately diagnose at which site in the body of the subject an abnormality occurs, in addition to the health condition of the subject.

### Embodiment 3

The following description will discuss still another embodiment of the present disclosure. Note that, for convenience, members having the same functions as those of the members described in the above embodiments will be given the same reference signs and descriptions thereof will not be repeated.

### (Configuration of information processing system 100b)

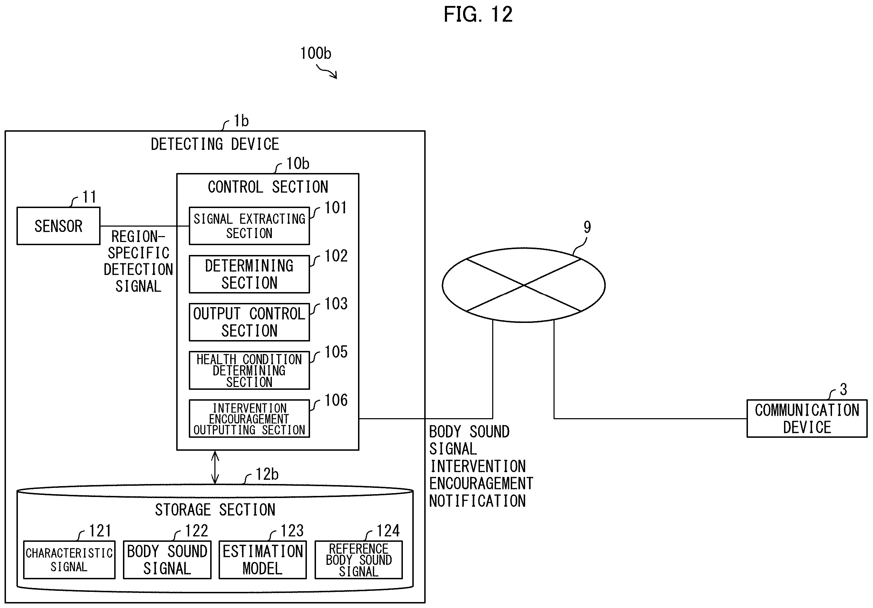

An information processing system 100b that has a function of estimating the health condition of a subject on the basis of a body sound signal is described with reference to Fig. 12. Fig. 12 is a functional block diagram illustrating an example of a configuration of the information processing system 100b. Note that Fig. 12 illustrates an example in which a sensor 11 that includes a plurality of detection regions outputs region-specific detection signals. However, the sensor 11 is not limited to this example. For example, in a case where the sensor 11 includes a single detection region, the sensor 11 may output a single detection signal, as illustrated in Fig. 5.

As illustrated in Fig. 12, the information processing system 100b may include a detecting device 1b (information processing device) and a communication device 3 (information processing device). The detecting device 1b includes the sensor 11, a control section 10b, and a storage section 12b. The control section 10b includes a health condition determining section 105, in addition to a signal extracting section 101, a determining section 102 (sleep determining section, posture determining section), and an output control section 103 (first output control section, second output control section). The control section 10b may further include an intervention encouragement outputting section 106. In the storage section 12b, a reference body sound signal 124 (described later) is stored.

The health condition determining section 105 determines the health condition of the subject by comparing the body sound signal of the subject with the given reference body sound signal 124 that is stored in the storage section 12b. Note, here, that the given reference body sound signal 124 may be a body sound signal that has been extracted in advance from a detection signal detected with regard to a healthy person. Alternatively, the given reference body sound signal 124 may be a body sound signal that has been extracted in advance from a detection signal detected with regard to a patient who is affected by a given disease. The health condition determining section 105 may determine the health condition of the subject on the basis of a difference between the body sound signal of the subject and the reference body sound signal 124 that has been extracted from the detection signal of the healthy person. Alternatively, the health condition determining section 105 may determine the health condition of the subject on the basis of a similarity between the body sound signal of the subject and the reference body sound signal 124 that has been extracted from the detection signal of the patient who is affected by the given disease.

For example, a disease such as chronic obstructive pulmonary disease (COPD) and bronchial asthma often exhibits an abnormality in an exhalation segment. There can be also a disease that exhibits a characteristic abnormality in an inhalation segment. Thus, the health condition determining section 105 may be capable of determining the inhalation segment and the exhalation segment, on the basis of a breathing vibration signal included in the detection signal of the subject. In this case, the health condition determining section 105 may determine the health condition of the subject by comparing the body sound signal of the subject and the given reference body sound signal with regard to each of the inhalation segment and the exhalation segment. By employing this configuration, it is possible to improve the determination accuracy of the information processing system 100b with regard to the health condition of the subject.

The health condition determining section 105 may determine that the subject is in an abnormal state, in at least one of the following cases (1) to (3).
(1) A lung sound signal that indicates a lung sound of the subject is attenuated or lost as compared with the given reference body sound signal.
(2) An abnormal sound signal that indicates an abnormal sound is mixed in the lung sound signal of the subject.
(3) An adventitious sound signal that indicates an adventitious sound is mixed in the lung sound signal of the subject.

In a case where the lung sound signal of the subject is attenuated or lost as compared with a lung sound signal that has been extracted from the given reference body sound signal 124, the health condition determining section 105 may determine that the subject is affected by at least one of pneumothorax, pleural effusion, and atelectasis. In a case where the abnormal sound signal is mixed in the lung sound signal of the subject, the health condition determining section 105 may determine that the subject is affected by at least one of chronic obstructive pulmonary disease and bronchial asthma.

The intervention encouragement outputting section 106 outputs an intervention encouragement notification that encourages a medical intervention with respect to the subject, in a case where the health condition determining section 105 has determined that the health condition of the subject is in the abnormal state. The intervention encouragement notification may notify (i) whether the medical intervention with respect to the subject is necessary or not and (ii) the greatness of the necessity of the medical intervention. For example, the intervention encouragement outputting section 106 may output, to the communication device 3, the intervention encouragement notification so as to encourage the medical intervention with respect to the subject by a medical expert and others.

A configuration in which the detecting device 1b includes the health condition determining section 105 and the intervention encouragement outputting section 106 has been described here as an example. However, the information processing system 100b is not limited to this configuration. For example, the communication device 3 may have functions similar to those of the health condition determining section 105 and the intervention encouragement outputting section 106.

### (Process carried out by information processing system 100b)

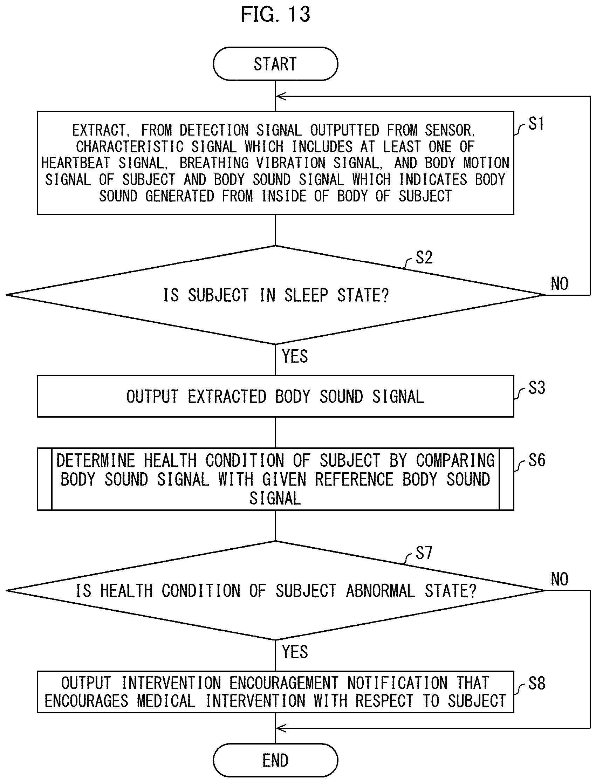

Next, a flow of a process carried out by the information processing system 100b is described with reference to Fig. 13. Fig. 13 is a flowchart illustrating an example of the flow of the process carried out by the information processing system 100b. A case where the determining section 102 of the information processing system 100b determines that the subject is in a sleep state is described here as an example. However, the information processing system 100b is not limited to this example. The determining section 102 of the information processing system 100b may be configured to determine that the subject is in a given posture.

The signal extracting section 101 extracts a characteristic signal and the body sound signal of the subject from the detection signal outputted from the sensor 11 (step S1: signal extracting step). The signal extracting section 101 may extract the characteristic signal and the body sound signal of the subject from each of the region-specific detection signals.

The determining section 102 determines that the subject is in the sleep state, on the basis of the characteristic signal (step S2: sleep determining step). In a case where it has been determined that the subject is not in the sleep state (NO in the step S2), the process returns to the step S 1.

In a case where it has been determined that the subject is in the sleep state (YES in the step S2), the output control section 103 (first output control section) outputs the body sound signal that has been extracted from each of the region-specific detection signals detected during a time period during which it has been determined that the subject is in the sleep state (step S3: first output controlling step).

The health condition determining section 105 determines the health condition of the subject by comparing the body sound signal that has been outputted with the given reference body sound signal 124 (step S6: health condition determining step). Note, here, that the information processing system 100b may be configured such that the output control section 103 outputs a determination result regarding the health condition of the subject. Note that a process carried out by the health condition determining section 105 in the step S6 will be described later with reference to a specific example.

In a case where it has been determined that the health condition of the subject is the abnormal state (YES in a step S7), the intervention encouragement outputting section 106 outputs the intervention encouragement notification (step S8: intervention encouragement outputting step). In a case where it has not been determined that the health condition of the subject is the abnormal state (NO in the step S7), the intervention encouragement outputting section 106 does not output the intervention encouragement notification.

### [Process carried out by health condition determining section 105]

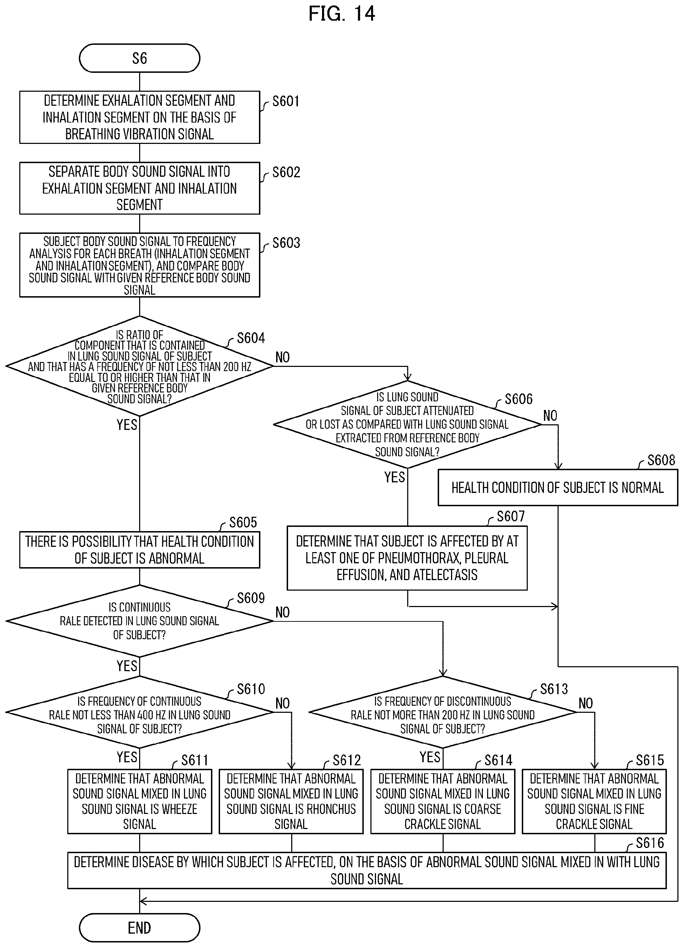

Next, an example of the process carried out by the health condition determining section 105 is described with reference to Fig. 14. Fig. 14 is a flowchart illustrating an example of a flow of a process of determining the health condition of the subject which process is carried out by the information processing system 100b.

The health condition determining section 105 determines the exhalation segment and the inhalation segment on the basis of the breathing vibration signal that has been extracted from the detection signal (step S601). Next, the health condition determining section 105 separates, into the exhalation segment and the inhalation segment, the body sound signal that has been extracted from the same detection signal and that has been outputted in the step S3 (step S602).

Next, the health condition determining section 105 subjects the body sound signal to a frequency analysis for each breath (including a pair of the exhalation segment and the inhalation segment), and compares the body sound signal with the given reference body sound signal 124 (step S603).

Next, the health condition determining section 105 determines whether a ratio of a component that is contained in the lung sound signal of the subject and that has a frequency of not less than 200 Hz is equal to or higher than that in the lung sound signal that has been extracted from the given reference body sound signal (step S604). In the case of YES in the step S604, the health condition determining section 105 determines that there is a possibility that the health condition of the subject is abnormal (step S605).

In the case of NO in the step S604, the health condition determining section 105 determines whether the lung sound signal of the subject is attenuated or lost as compared with the lung sound signal that has been extracted from the reference body sound signal 124 (step S606).

In a case where the lung sound signal of the subject is attenuated or lost as compared with the lung sound signal that has been extracted from the reference body sound signal 124 (YES in the step S606), the health condition determining section 105 determines that the subject is affected by at least one of pneumothorax, pleural effusion, and atelectasis (step S607). In a case where the lung sound signal of the subject is not attenuated and is also not lost as compared with the lung sound signal that has been extracted from the reference body sound signal 124 (NO in the step S606), the health condition determining section 105 determines that the health condition of the subject is normal (no disease) (step S608).

The health condition determining section 105 can also carry out the following processes in steps S609 to S616.

The health condition determining section 105 determines whether a continuous rale is detected in the lung sound signal of the subject whose the health condition has been determined as being abnormal (step S609). In a case where the continuous rale is detected in the lung sound signal of the subject (YES in the step 609) and in a case where the frequency of the continuous rale is not less than 400 Hz (YES in a step 610), the health condition determining section 105 determines that the abnormal sound signal that is mixed in the lung sound of the subject is a wheeze signal (step S611). In a case where the continuous rale that is detected in the lung sound signal of the subject has a frequency of less than 400 Hz (NO in the step 610), the health condition determining section 105 determines that the abnormal sound signal that is mixed in the lung sound of the subject is a rhonchus signal (step S612).

In a case where the continuous rale is not detected in the lung sound signal of the subject (NO in the step 609) and in a case where the frequency of a discontinuous rale is not more than 200 Hz (YES in a step 613), the health condition determining section 105 determines that the abnormal sound signal that is mixed in the lung sound of the subject is a coarse crackle signal (step S614). In a case where the discontinuous rale that is detected in the lung sound signal of the subject has a frequency of higher than 200 Hz (NO in the step 613), the health condition determining section 105 determines that the abnormal sound signal that is mixed in the lung sound of the subject is a fine crackle signal (step S615).

The health condition determining section 105 determines a disease by which the subject is affected, on the basis of the abnormal sound signal mixed in with the lung sound signal (step S616).

### (Example display of body sound signal and intervention encouragement notification)

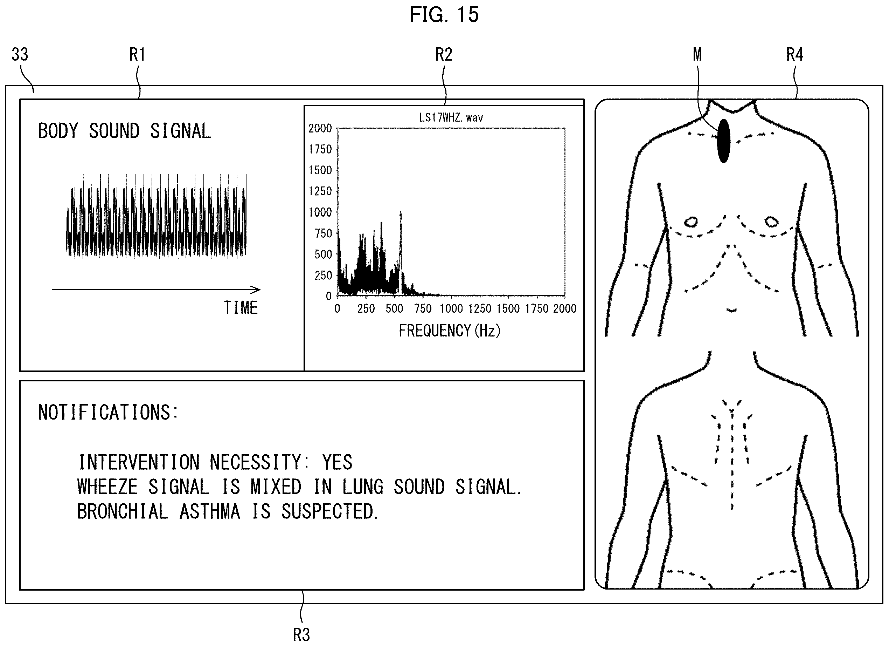

A screen display that is shown on the display section 33 in a case where the communication device 3 of the information processing system 100b has obtained the body sound signal and the intervention encouragement notification is described with reference to Fig. 15. Fig. 15 is a view illustrating an example of a display screen displayed on the display section 33 of the communication device 3 of the information processing system 100b.

As illustrated in Fig. 15, a region R1 in which waveform data of the body sound signal of the subject is displayed and a region R3 in which the intervention encouragement notification is displayed may be displayed on the display section 33. In the region R3 illustrated in Fig. 15, the intervention encouragement notification "Intervention necessity: Yes. Wheeze signal is mixed in lung sound signal. Bronchial asthma is suspected." is displayed.

Note that the display section 33 may be configured such that a region R2 in which a graph or the like that shows the frequency characteristic of the body sound signal is displayed is displayed. The graph that shows the frequency characteristic may be, for example, a graph that shows the frequency (Hz) on the horizontal axis and a power value (dB) on the vertical axis, as illustrated in Fig. 15. Alternatively, as an alternative to the graph that shows the frequency characteristic, a sonogram that shows a time (second) on the horizontal axis and the frequency (Hz) on the vertical axis may be, for example, displayed.

The information processing system 100b may be configured to further include the site estimating section 104 of the information processing system 100a. In this case, the display screen may further include a region R4 in which abnormal site information regarding the subject can be displayed, as illustrated in Fig. 15. In an example illustrated in Fig. 15, an abnormal sound occurring site is shown as an elliptical mark M, in a figure that is included in the region R4 and that simulates the front and back of the body of the subject.

For example, it is possible for the medical expert and others to appropriately determine the health condition of the subject, the type and site of the disease by which the subject is affected, and the necessity of the medical intervention with respect to the subject, with reference to a display displayed on the communication device 3.

### Embodiment 4

The following description will discuss another embodiment of the present disclosure. Note that, for convenience, members having the same functions as those of the members described in the above embodiments will be given the same reference signs and descriptions thereof will not be repeated.

### (Configuration of information processing system 100c)

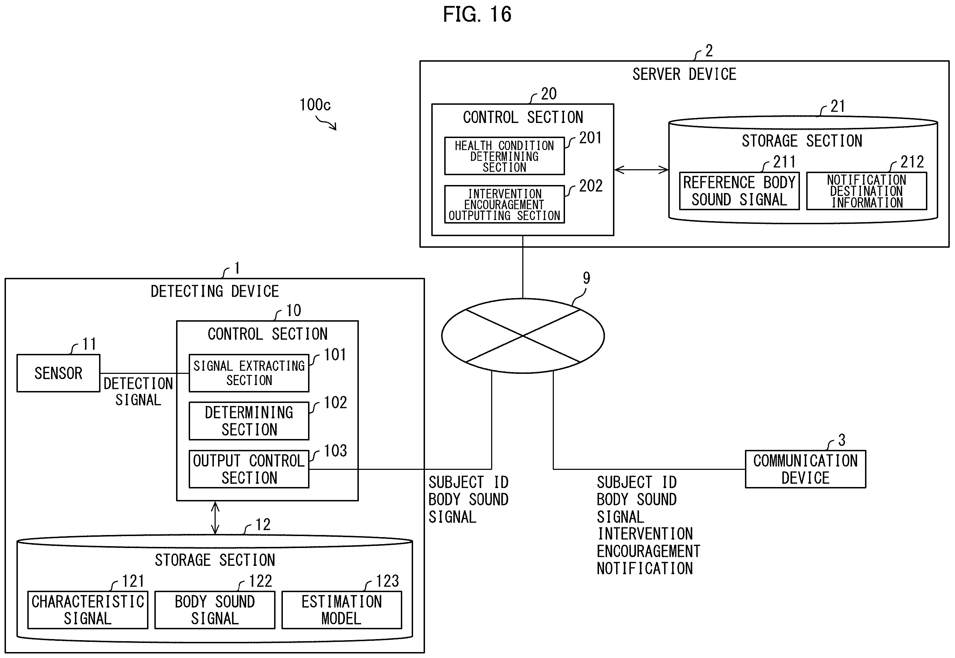

A server device 2 (information processing device) that is communicably connected to a detecting device 1 (information processing device) and a communication device 3 (information processing device) may be configured to have functions similar to those of the health condition determining section 105 and the intervention encouragement outputting section 106 of the information processing system 100b. Such a configuration is described with reference to Fig. 16. Fig. 16 is a functional block diagram illustrating an example of a configuration of an information processing system 100c.

As illustrated in Fig. 16, the information processing system 100c may include the detecting device 1, the communication device 3, and the server device 2. The information processing system 100c may include a single server device 2 or a plurality of server devices 2. The server device 2 may be a server device realized by cloud computing as an example.

The server device 2 includes a control section 20 and a storage section 21. The control section 20 may be a CPU in an example. The control section 20 carries out various functions by reading a control program that is software stored in the storage section 21 and loading the control program in a memory such as a RAM.

As illustrated in Fig. 16, the control section 20 includes a health condition determining section 201 and an intervention encouragement outputting section 202. In the storage section 21, a reference body sound signal 211 and notification destination information 212 that indicates a destination to which an intervention encouragement signal is outputted are stored. The reference body sound signal 211 may be the same as the reference body sound signal 124 of the information processing system 100b.

The health condition determining section 201 has a function similar to that of the health condition determining section 105 of the information processing system 100b. The intervention encouragement outputting section 202 has a function similar to that of the intervention encouragement outputting section 106 of the information processing system 100b.

In the information processing system 100c, a body sound signal that has been extracted from a detection signal detected during a time period during which the state of a subject is at least one of a sleep state and a given posture is first outputted from the detecting device 1. At this time, a subject ID that is identification information unique to the subject may be associated with the body sound signal that is outputted. Note that Fig. 16 illustrates an example in which a sensor 11 that includes a single detection region outputs the detection signal. However, the sensor 11 is not limited to this example. For example, in a case where the sensor 11 includes a plurality of detection regions, the sensor 11 may output region-specific detection signals, as illustrated in Fig. 10.

Next, the server device 2 obtains the body sound signal, determines the health condition of the subject, and outputs an intervention encouragement notification. The communication device 3 obtains the body sound signal that has been outputted from the detecting device 1 and the intervention encouragement notification that has been outputted from the server device 2, and displays the body sound signal and the intervention encouragement notification on a display section 33.

### Embodiment 5

The following description will discuss another embodiment of the present disclosure. Note that, for convenience, members having the same functions as those of the members described in the above embodiments will be given the same reference signs and descriptions thereof will not be repeated.

In the present embodiment, a part of the function of the detecting device 1b included in the information processing system 100b in accordance with Embodiment 3 is shared by a server device.

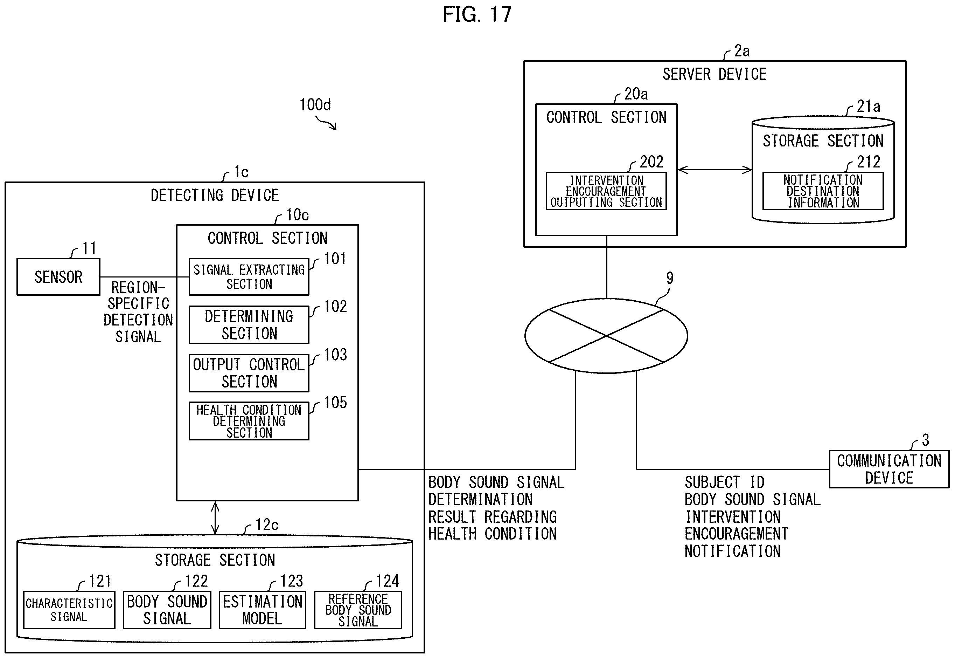

Fig. 17 is a view illustrating an example of a configuration of an information processing system 100d in accordance with the present embodiment. As illustrated in Fig. 17, the information processing system 100d includes a server device 2a (information processing device), a detecting device 1c, and a communication device 3.

The server device 2a includes a control section 20a and a storage section 21a. The control section 20a may be a CPU in an example. The control section 20a carries out various functions by reading a control program that is software stored in the storage section 21a and loading the control program in a memory such as a RAM. The control section 20a includes an intervention encouragement outputting section 202. In the storage section 21a, notification destination information 212 that indicates a destination to which an intervention encouragement signal is outputted is stored.

The detecting device 1c includes a control section 10c and a storage section 12c. The control section 10c may be a CPU in an example. The control section 10c carries out various functions by reading a control program that is software stored in the storage section 12c and loading the control program in a memory such as a RAM. The control section 10c includes a signal extracting section 101, a determining section 102, an output control section 103, and a health condition determining section 105. In the storage section 12c, a characteristic signal 121, a body sound signal 122, an estimation model 123, and a reference body sound signal 124 are stored.

In a case where this configuration is employed, the detecting device 1c carries out a process of determining the health condition of a subject, and outputs a determination result to the server device 2a. Next, the server device 2a obtains the determination result regarding the health condition of the subject from the detecting device 1c. In a case where the determination result thus obtained is a determination result that indicates that the health condition of the subject is an abnormal state, the server device 2a outputs an intervention encouragement notification that encourages a medical intervention with respect to the subject.

### Embodiment 6

The following description will discuss another embodiment of the present disclosure. Note that, for convenience, members having the same functions as those of the members described in the above embodiments will be given the same reference signs and descriptions thereof will not be repeated.

The detecting device 1 in accordance with Embodiment 1 has been described as including the sensor 11, the control section 10, and the storage section 12. However, the sensor 11, the control section 10, and the storage section 12 do not need to be configured as an integral device. In the present embodiment, the sensor 11, the control section 10, and the storage section 12 are configured as separate devices.

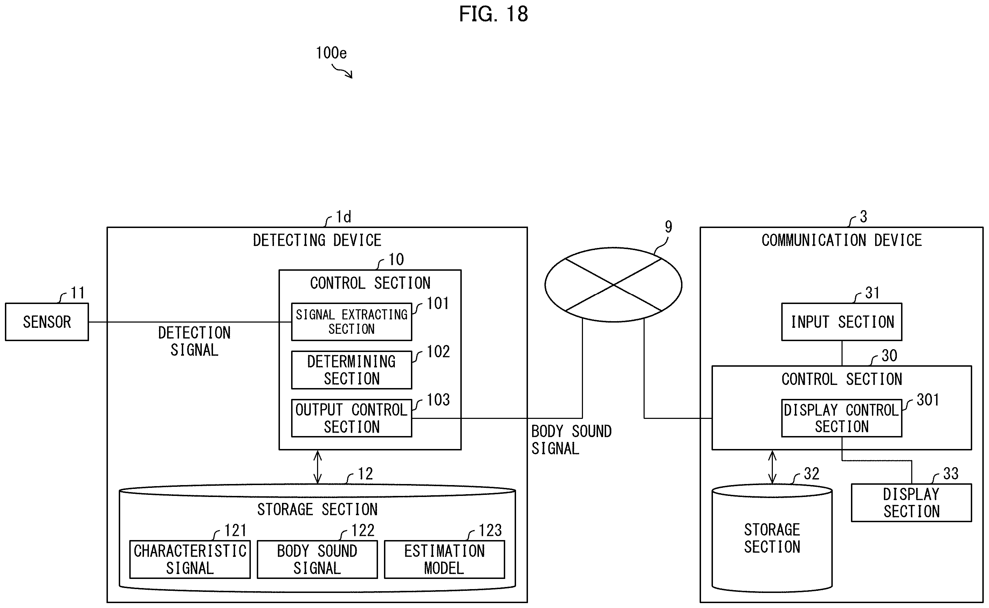

Fig. 18 is a functional block diagram illustrating an example of a configuration of an information processing system 100e in accordance with the present embodiment. The information processing system 100e includes a sensor 11, a detection signal analyzing device 1d (information processing device), and a communication device 3.

The detection signal analyzing device 1d includes a control section 10 and a storage section 12. The detection signal analyzing device 1d may be a computer that is communicably connected to the sensor 11. The sensor 11 and the detection signal analyzing device 1d may be directly connected to each other or may be alternatively communicably connected to each other with a LAN therebetween.

The configuration of the information processing system 100e is not limited to the example illustrated in Fig. 18, and each of the detecting devices 1a, 1b, and 1c can be separated into the sensor 11 and a detection signal analyzing device (information processing device) that includes a corresponding one of the control sections 10a, 10b, and 10c and a corresponding one of the storage sections 12a, 12b, and 12c.

### [Software implementation example]

The function of each of the detecting devices 1, 1a, 1b, and 1c, the detection signal analyzing device 1d, the server device 2, and the communication device 3 (hereinafter, referred to as "device") can be realized by a program for causing a computer to function as the device, the program causing the computer to function as each control block (in particular, each section included in the control sections 10, 10a, 10b, 10c, 20, and 30) of the device.

In this case, the device includes, as hardware for executing the program, a computer which includes at least one control device (e.g., processor) and at least one storage device (e.g., memory). Each function described in each of the foregoing embodiments can be realized by executing the program with use of the control device and the storage device.

The program may be recorded in one or more non-transitory computer-readable recording mediums. This recording medium may or may not be provided to the device. In the latter case, the program may be made available to the device via any wired or wireless transmission medium.

Further, a part or all of the function of each control block can be realized by a logic circuit. For example, the present disclosure encompasses, in its scope, an integrated circuit in which the logic circuit that functions as each control block is formed. As another alternative, the function of each control block can be realized by, for example, a quantum computer.

Each process described in each of the foregoing embodiments may be carried out by artificial intelligence (AI). In this case, the AI may operate in the control device, or may operate in another device (e.g., an edge computer or a cloud server).

The present disclosure is not limited to the embodiments, but can be altered variously by a skilled person in the art within the scope of the claims. The present disclosure also encompasses, in its technical scope, any embodiment derived by appropriately combining technical means disclosed in differing embodiments.

Aspects of the present invention can also be expressed as follows:
An information processing system in accordance with a first aspect of the present disclosure is an information processing system including: a signal extracting section that extracts, from a detection signal outputted from a sensor, (i) a characteristic signal which includes at least one of a heartbeat signal that indicates a heartbeat of a subject, a breathing vibration signal that indicates a breathing vibration of the subject, and a body motion signal that indicates a body motion of the subject and (ii) a body sound signal which indicates a body sound that is generated from an inside of a body of the subject, the sensor detecting, at a position at which the sensor is not in contact with the subject, vibrations generated by the subject; a sleep determining section that determines that the subject is in a sleep state, on the basis of the characteristic signal that has been extracted; and a first output control section that outputs the body sound signal during a time period during which it has been determined that the subject is in the sleep state, in a case where it has been determined that the subject is in the sleep state.

The information processing system in accordance with a second aspect of the present disclosure may be configured such that, in the first aspect, the sleep determining section estimates the sleep state by inputting, into an estimation model, the characteristic signal that has been extracted by the signal extracting section, the estimation model having been trained by machine learning with use of training data in which the characteristic signal is an explanatory variable and the sleep state is a response variable.

An information processing system in accordance with a third aspect of the present invention is an information processing system including: a signal extracting section that extracts, from a detection signal outputted from a sensor, (i) a characteristic signal which includes at least one of a heartbeat signal that indicates a heartbeat of a subject, a breathing vibration signal that indicates a breathing vibration of the subject, and a body motion signal that indicates a body motion of the subject and (ii) a body sound signal which indicates a body sound that is generated from an inside of a body of the subject, the sensor detecting, at a position at which the sensor is not in contact with the subject, vibrations generated by the subject; a posture determining section that determines that the subject is in a given posture, on the basis of at least one of the detection signal and the characteristic signal that has been extracted; and a second output control section that outputs the body sound signal during a time period during which it has been determined that the subject is in the given posture, in a case where it has been determined that the subject is in the given posture.

The information processing system in accordance with a fourth aspect of the present disclosure may be configured such that, in the third aspect, the posture determining section estimates a probability that the subject is in the given posture, by inputting, into an estimation model, at least one of the detection signal that has been detected by the sensor and the characteristic signal that has been extracted by the signal extracting section, the estimation model having been trained by machine learning with use of training data in which at least one of the detection signal and the characteristic signal is an explanatory variable and a posture of the subject is a response variable.

The information processing system in accordance with a fifth aspect of the present disclosure may be such that, in the third or fourth aspect, the given posture is at least one of a right lateral decubitus position, a left lateral decubitus position, a supine position, a prone position, and a sitting position.

The information processing system in accordance with a sixth aspect of the present disclosure may be such that, in any one of the first through fifth aspects, the sensor includes a plurality of detection regions each of which outputs the detection signal, and the signal extracting section extracts the characteristic signal and the body sound signal from each of region-specific detection signals that are outputted from the respective plurality of detection regions.

The information processing system in accordance with a seventh aspect of the present disclosure may further include, in the sixth aspect, a site estimating section that estimates an abnormal sound occurring site in the body of the subject, on the basis of the body sound signal that has been extracted from each of the region-specific detection signals.

The information processing system in accordance with an eighth aspect of the present disclosure may be such that, in any one of the first through seventh aspects, the sensor obtains at least one of the body sound signal that has a frequency of not less than 100 Hz, the heartbeat signal that has a frequency of not more than 20 Hz, the breathing vibration signal, the body motion signal and the rhonchus signal that has a frequency of not less than 100 Hz.

The information processing system in accordance with a ninth aspect of the present disclosure may be configured such that, in any one of the first through eighth aspects, the sensor has a thin plate-like shape and is set on a bed on which the subject lies down.

The information processing system in accordance with a tenth aspect of the present disclosure may be such that, in any one of the first through ninth aspects, the body sound signal includes at least one of a lung sound signal that indicates a lung sound of the subject, a blood flow sound signal that indicates a blood flow sound of the subject, a heart sound signal that indicates a heart sound of the subject, a bowel sound signal that indicates a bowel sound of the subject, and a peristaltic sound signal that indicates a peristaltic sound of the subject.

The information processing system in accordance with an eleventh aspect of the present disclosure may further include, in any one of the first through tenth aspects, a health condition determining section that determines a health condition of the subject by comparing the body sound signal and a given reference body sound signal.

The information processing system in accordance with a twelfth aspect of the present disclosure may be such that, in the eleventh aspect, the health condition determining section is capable of determining an inhalation segment and an exhalation segment on the basis of the breathing vibration signal that is included in the detection signal, and determines the health condition of the subject by comparing the body sound signal and the given reference body sound signal with regard to each of the inhalation segment and the exhalation segment.

The information processing system in accordance with a thirteenth aspect of the present disclosure may be such that, in the eleventh or twelfth aspect, the health condition determining section determines that the subject is in an abnormal state, in at least one of (1) a case where a lung sound signal that indicates a lung sound of the subject is attenuated or lost as compared with the given reference body sound signal, (2) a case where an abnormal sound signal that indicates an abnormal sound is mixed in the lung sound signal, and (3) a case where an adventitious sound signal that indicates an adventitious sound is mixed in the lung sound signal, and the adventitious sound includes at least one of a coarse crackle, a fine crackle, a wheeze, a rhonchus, a stridor, a squawk, and a pleural friction rub of the subject.

The information processing system in accordance with a fourteenth aspect of the present disclosure may be such that, in the thirteenth aspect, the health condition determining section determines that the subject is affected by at least one of pneumothorax, pleural effusion, and atelectasis, in a case where the lung sound signal is attenuated or lost as compared with a lung sound signal that has been extracted from the given reference body sound signal, and determines that the subject is affected by at least one of chronic obstructive pulmonary disease and bronchial asthma, in a case where the abnormal sound signal is mixed in the lung sound signal.

The information processing system in accordance with a fifteenth aspect of the present disclosure may further include, in the thirteenth or fourteenth aspect, an intervention encouragement outputting section that outputs an intervention encouragement notification which encourages a medical intervention with respect to the subject, in a case where the health condition determining section has determined that the health condition of the subject is the abnormal state.

A detecting device in accordance with a sixteenth aspect of the present disclosure includes: a sensor that detects, at a position at which the sensor is not in contact with a subject, vibrations generated by the subject; a signal extracting section that extracts, from a detection signal outputted from the sensor, (i) a characteristic signal which includes at least one of a heartbeat signal that indicates a heartbeat of the subject, a breathing vibration signal that indicates a breathing vibration of the subject, and a body motion signal that indicates a body motion of the subject and (ii) a body sound signal which indicates a body sound that is generated from an inside of a body of the subject; a sleep determining section that determines that the subject is in a sleep state, on the basis of the characteristic signal that has been extracted; and a first output control section that outputs the body sound signal during a time period during which it has been determined that the subject is in the sleep state, in a case where it has been determined that the subject is in the sleep state.

A detecting device in accordance with a seventeenth aspect of the present disclosure includes: a sensor that detects, at a position at which the sensor is not in contact with a subject, vibrations generated by the subject; a signal extracting section that extracts, from a detection signal outputted from the sensor, (i) a characteristic signal which includes at least one of a heartbeat signal that indicates a heartbeat of the subject, a breathing vibration signal that indicates a breathing vibration of the subject, and a body motion signal that indicates a body motion of the subject and (ii) a body sound signal which indicates a body sound that is generated from an inside of a body of the subject; a posture determining section that determines that the subject is in a given posture, on the basis of at least one of the detection signal and the characteristic signal that has been extracted; and a second output control section that outputs the body sound signal during a time period during which it has been determined that the subject is in the given posture, in a case where it has been determined that the subject is in the given posture.

The detecting device in accordance with an eighteenth aspect of the present disclosure may further include, in the sixteenth or seventeenth aspect, a health condition determining section that determines a health condition of the subject by comparing the body sound signal and a given reference body sound signal.

The detecting device in accordance with a nineteenth aspect of the present disclosure may further include, in the eighteenth aspect, an intervention encouragement outputting section that outputs an intervention encouragement notification which encourages a medical intervention with respect to the subject, in a case where the health condition determining section has determined that the health condition of the subject is an abnormal state.

A server device in accordance with a twentieth aspect of the present disclosure includes comprising: a health condition determining section that obtains the body sound signal which has been outputted from the detecting device described in the sixteenth or seventeenth aspect and that determines a health condition of the subject by comparing the body sound signal and a given reference body sound signal; and an intervention encouragement outputting section that outputs an intervention encouragement notification which encourages a medical intervention with respect to the subject, in a case where the health condition determining section has determined that the health condition of the subject is an abnormal state.

A server device in accordance with a twenty-first aspect of the present disclosure includes an intervention encouragement outputting section that obtains a determination result which has been outputted from the detecting device described in the eighteenth aspect and which relates to the health condition and that outputs an intervention encouragement notification which encourages a medical intervention with respect to the subject, in a case where the determination result that has been obtained indicates that the health condition of the subject is an abnormal state.

An information processing device in accordance with a twenty-second aspect of the present disclosure includes: a signal extracting section that obtains a detection signal outputted from a sensor which detects, at a position at which the sensor is not in contact with a subject, vibrations generated by the subject and that extracts, from the detection signal, (i) a characteristic signal which includes at least one of a heartbeat signal that indicates a heartbeat of the subject, a breathing vibration signal that indicates a breathing vibration of the subject, and a body motion signal that indicates a body motion of the subject and (ii) a body sound signal which indicates a body sound that is generated from an inside of a body of the subject; a sleep determining section that determines that the subject is in a sleep state, on the basis of the characteristic signal that has been extracted; and a first output control section that outputs the body sound signal during a time period during which it has been determined that the subject is in the sleep state, in a case where it has been determined that the subject is in the sleep state.

An information processing device in accordance with a twenty-third aspect of the present disclosure includes: a signal extracting section that obtains a detection signal outputted from a sensor which detects, at a position at which the sensor is not in contact with a subject, vibrations generated by the subject and that extracts, from the detection signal, (i) a characteristic signal which includes at least one of a heartbeat signal that indicates a heartbeat of the subject, a breathing vibration signal that indicates a breathing vibration of the subject, and a body motion signal that indicates a body motion of the subject and (ii) a body sound signal which indicates a body sound that is generated from an inside of a body of the subject; a posture determining section that determines that the subject is in a given posture, on the basis of at least one of the detection signal and the characteristic signal that has been extracted; and a second output control section that outputs the body sound signal during a time period during which it has been determined that the subject is in the given posture, in a case where it has been determined that the subject is in the given posture.

A control method in accordance with a twenty-fourth aspect of the present disclosure is a control method which is carried out by one or more information processing devices, including: a signal extracting step of extracting, from a detection signal outputted from a sensor, (i) a characteristic signal which includes at least one of a heartbeat signal that indicates a heartbeat of a subject, a breathing vibration signal that indicates a breathing vibration of the subject, and a body motion signal that indicates a body motion of the subject and (ii) a body sound signal which indicates a body sound that is generated from an inside of a body of the subject, the sensor detecting, at a position at which the sensor is not in contact with the subject, vibrations generated by the subject; a sleep determining step of determining that the subject is in a sleep state, on the basis of the characteristic signal that has been extracted; and a first output controlling step of outputting the body sound signal during a time period during which it has been determined that the subject is in the sleep state, in a case where it has been determined that the subject is in the sleep state.

A control method in accordance with a twenty-fifth aspect of the present disclosure is a control method which is carried out by one or more information processing devices, including: a signal extracting step of extracting, from a detection signal outputted from a sensor, (i) a characteristic signal which includes at least one of a heartbeat signal that indicates a heartbeat of a subject, a breathing vibration signal that indicates a breathing vibration of the subject, and a body motion signal that indicates a body motion of the subject and (ii) a body sound signal which indicates a body sound that is generated from an inside of a body of the subject, the sensor detecting, at a position at which the sensor is not in contact with the subject, vibrations generated by the subject; a posture determining step of determining that the subject is in a given posture, on the basis of at least one of the detection signal and the characteristic signal that has been extracted; and a second output controlling step of outputting the body sound signal during a time period during which it has been determined that the subject is in the given posture, in a case where it has been determined that the subject is in the given posture.

A control method in accordance with a twenty-sixth aspect of the present disclosure is a control method which is carried out by one or more server devices, including: a health condition determining step of obtaining the body sound signal which has been outputted from the one or more information processing devices each of which carries out the control method described in the twenty-fourth or twenty-fifth aspect and determining a health condition of the subject by comparing the body sound signal and a given reference body sound signal; and an intervention encouragement outputting step of outputting an intervention encouragement notification which encourages a medical intervention with respect to the subject, in a case where it has been determined, in the health condition determining step, that the health condition of the subject is an abnormal state.

A program in accordance with a twenty-seventh aspect of the present disclosure is a program for causing a computer to function as the information processing system described in the first aspect, the program causing the computer to function as the signal extracting section, the sleep determining section, and the first output control section.

A program in accordance with a twenty-eighth aspect of the present disclosure is a program for causing a computer to function as the information processing system described in the third aspect, the program causing the computer to function as the signal extracting section, the posture determining section, and the second output control section.

A program in accordance with a twenty-ninth aspect of the present disclosure is a program for causing a computer to function as the server device described in the twentieth aspect, the program causing the computer to function as the health condition determining section and the intervention encouragement outputting section.

A program in accordance with a thirtieth aspect of the present disclosure is a program for causing a computer to function as the server device described in the twenty-first aspect, the program causing the computer to function as the intervention encouragement outputting section.

### [Supplementary note]

An information processing system in accordance with a first aspect of the present disclosure is an information processing system including: a signal extracting section that extracts, from a detection signal outputted from a sensor, (i) a characteristic signal which includes at least one of a heartbeat signal that indicates a heartbeat of a subject, a breathing vibration signal that indicates a breathing vibration of the subject, a body motion signal that indicates a body motion of the subject, and a rhonchus signal that indicates a rhonchus of the subject and (ii) a body sound signal which indicates a body sound that is generated from an inside of a body of the subject, the sensor detecting, at a position at which the sensor is not in contact with the subject, vibrations generated by the subject; a sleep determining section that determines that the subject is in a sleep state, on the basis of the characteristic signal that has been extracted; and a first output control section that outputs the body sound signal which has been extracted from the detection signal detected during a time period during which it has been determined that the subject is in the sleep state, in a case where it has been determined that the subject is in the sleep state.

An information processing system in accordance with a second aspect of the present disclosure is an information processing system including: a signal extracting section that extracts, from a detection signal outputted from a sensor, (i) a characteristic signal which includes at least one of a heartbeat signal that indicates a heartbeat of a subject, a breathing vibration signal that indicates a breathing vibration of the subject, a body motion signal that indicates a body motion of the subject, and a rhonchus signal that indicates a rhonchus of the subject and (ii) a body sound signal which indicates a body sound that is generated from an inside of a body of the subject, the sensor detecting, at a position at which the sensor is not in contact with the subject, vibrations generated by the subject; a posture determining section that determines that the subject is in a given posture, on the basis of at least one of the detection signal and the characteristic signal that has been extracted; and a second output control section that outputs the body sound signal which has been extracted from the detection signal detected during a time period during which it has been determined that the subject is in the given posture, in a case where it has been determined that the subject is in the given posture.

A detecting device in accordance with a third aspect of the present disclosure includes: a sensor that detects, at a position at which the sensor is not in contact with a subject, vibrations generated by the subject; a signal extracting section that extracts, from a detection signal outputted from the sensor, (i) a characteristic signal which includes at least one of a heartbeat signal that indicates a heartbeat of the subject, a breathing vibration signal that indicates a breathing vibration of the subject, a body motion signal that indicates a body motion of the subject, and a rhonchus signal that indicates a rhonchus of the subject and (ii) a body sound signal which indicates a body sound that is generated from an inside of a body of the subject; a sleep determining section that determines that the subject is in a sleep state, on the basis of the characteristic signal that has been extracted; and a first output control section that outputs the body sound signal which has been extracted from the detection signal detected during a time period during which it has been determined that the subject is in the sleep state, in a case where it has been determined that the subject is in the sleep state.

A detecting device in accordance with a fourth aspect of the present disclosure includes: a sensor that detects, at a position at which the sensor is not in contact with a subject, vibrations generated by the subject; a signal extracting section that extracts, from a detection signal outputted from the sensor, (i) a characteristic signal which includes at least one of a heartbeat signal that indicates a heartbeat of the subject, a breathing vibration signal that indicates a breathing vibration of the subject, a body motion signal that indicates a body motion of the subject, and a rhonchus signal that indicates a rhonchus of the subject and (ii) a body sound signal which indicates a body sound that is generated from an inside of a body of the subject; a posture determining section that determines that the subject is in a given posture, on the basis of at least one of the detection signal and the characteristic signal that has been extracted; and a second output control section that outputs the body sound signal which has been extracted from the detection signal detected during a time period during which it has been determined that the subject is in the given posture, in a case where it has been determined that the subject is in the given posture.

A server device in accordance with a fifth aspect of the present disclosure is a server device including: a health condition determining section that obtains the body sound signal which has been outputted from the detecting device in accordance with the third or fourth aspect and that determines a health condition of the subject by comparing the body sound signal and a given reference body sound signal; and an intervention encouragement outputting section that outputs an intervention encouragement notification which encourages a medical intervention with respect to the subject, in a case where the health condition determining section has determined that the health condition of the subject is an abnormal state.

An information processing device in accordance with a sixth aspect of the present disclosure includes: a signal extracting section that obtains a detection signal outputted from a sensor which detects, at a position at which the sensor is not in contact with a subject, vibrations generated by the subject and that extracts, from the detection signal, (i) a characteristic signal which includes at least one of a heartbeat signal that indicates a heartbeat of the subject, a breathing vibration signal that indicates a breathing vibration of the subject, a body motion signal that indicates a body motion of the subject, and a rhonchus signal that indicates a rhonchus of the subject and (ii) a body sound signal which indicates a body sound that is generated from an inside of a body of the subject; a sleep determining section that determines that the subject is in a sleep state, on the basis of the characteristic signal that has been extracted; and a first output control section that outputs the body sound signal which has been extracted from the detection signal detected during a time period during which it has been determined that the subject is in the sleep state, in a case where it has been determined that the subject is in the sleep state.

An information processing device in accordance with a seventh aspect of the present disclosure includes: a signal extracting section that obtains a detection signal outputted from a sensor which detects, at a position at which the sensor is not in contact with a subject, vibrations generated by the subject and that extracts, from the detection signal, (i) a characteristic signal which includes at least one of a heartbeat signal that indicates a heartbeat of the subject, a breathing vibration signal that indicates a breathing vibration of the subject, a body motion signal that indicates a body motion of the subject, and a rhonchus signal that indicates a rhonchus of the subject and (ii) a body sound signal which indicates a body sound that is generated from an inside of a body of the subject; a posture determining section that determines that the subject is in a given posture, on the basis of at least one of the detection signal and the characteristic signal that has been extracted; and a second output control section that outputs the body sound signal which has been extracted from the detection signal detected during a time period during which it has been determined that the subject is in the given posture, in a case where it has been determined that the subject is in the given posture.

A control method in accordance with an eighth aspect of the present disclosure is a control method which is carried out by one or more information processing devices, including: a signal extracting step of extracting, from a detection signal outputted from a sensor, (i) a characteristic signal which includes at least one of a heartbeat signal that indicates a heartbeat of a subject, a breathing vibration signal that indicates a breathing vibration of the subject, a body motion signal that indicates a body motion of the subject, and a rhonchus signal that indicates a rhonchus of the subject and (ii) a body sound signal which indicates a body sound that is generated from an inside of a body of the subject, the sensor detecting, at a position at which the sensor is not in contact with the subject, vibrations generated by the subject; a sleep determining step of determining that the subject is in a sleep state, on the basis of the characteristic signal that has been extracted; and a first output controlling step of outputting the body sound signal which has been extracted from the detection signal detected during a time period during which it has been determined that the subject is in the sleep state, in a case where it has been determined that the subject is in the sleep state.

A control method in accordance with a ninth aspect of the present disclosure is a control method which is carried out by one or more information processing devices, including: a signal extracting step of extracting, from a detection signal outputted from a sensor, (i) a characteristic signal which includes at least one of a heartbeat signal that indicates a heartbeat of a subject, a breathing vibration signal that indicates a breathing vibration of the subject, a body motion signal that indicates a body motion of the subject, and a rhonchus signal that indicates a rhonchus of the subject and (ii) a body sound signal which indicates a body sound that is generated from an inside of a body of the subject, the sensor detecting, at a position at which the sensor is not in contact with the subject, vibrations generated by the subject; a posture determining step of determining that the subject is in a given posture, on the basis of at least one of the detection signal and the characteristic signal that has been extracted; and a second output controlling step of outputting the body sound signal which has been extracted from the detection signal detected during a time period during which it has been determined that the subject is in the given posture, in a case where it has been determined that the subject is in the given posture.

A control method in accordance with a tenth aspect of the present disclosure is a control method which is carried out by one or more server devices, including: a health condition determining step of obtaining the body sound signal which has been outputted from the one or more information processing devices each of which carries out the control method in accordance with the eighth or ninth aspect and determining a health condition of the subject by comparing the body sound signal and a given reference body sound signal; and an intervention encouragement outputting step of outputting an intervention encouragement notification which encourages a medical intervention with respect to the subject, in a case where it has been determined, in the health condition determining step, that the health condition of the subject is an abnormal state.

The information processing systems, the detecting devices, the server devices, and the information processing devices in accordance with the aspects of the present disclosure may be realized by a computer. In this case, the present disclosure also encompasses, in its scope, (i) a control program for the information processing systems, the detecting devices, the server devices, and the information processing devices, the control program causing the computer to realize the information processing systems, the detecting devices, and the server devices by causing the computer to operate as each section (software element) of the information processing systems, the detecting devices, the server devices, and the information processing devices and (ii) a computer-readable recording medium in which the control program is recorded.

### Examples

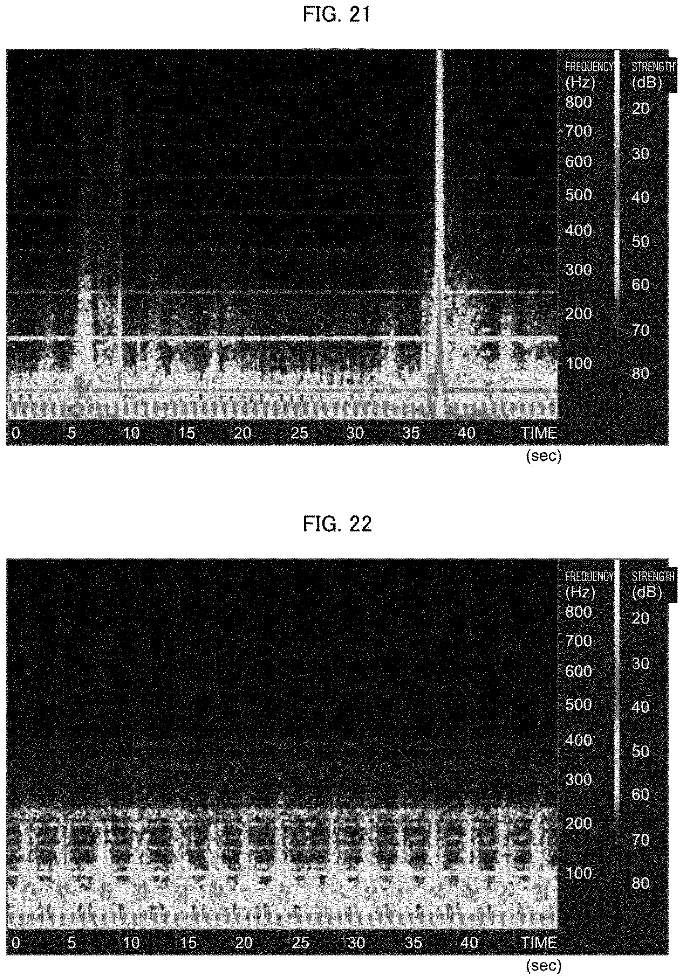

The following description will discuss examples of the present disclosure with reference to Figs. 21 to 36. Note that, in a sonogram shown in each of Figs. 21 to 29 and Figs. 33 to 36, the horizontal axis shows a time (second) and the vertical axis shows a frequency (Hz).

### <Body sound signal corresponding to waking/sleeping>

In each of Figs. 21 and 22, shown is an example of a body sound signal (including a lung sound signal) that is extracted from a detection signal outputted from a sensor 11 which detects, at a position at which the sensor 11 is not in contact with a subject (healthy person), vibrations generated by the subject in an awake state or a sleep state. Fig. 21 is a sonogram showing a body sound signal of a healthy person during awaking (immediately after the healthy person went to bed). Fig. 22 is a sonogram showing an example of a body sound signal of the healthy person during sleeping. In a case where the subject was awaking, breath sounds were louder (i.e., strength was higher) than those during sleeping, breathing rhythm was not steady, and noise derived from body motions was occasionally (e.g., around 39 seconds) detected, as shown in the sonogram shown in Fig. 21. In a case where the subject was sleeping, the breath sounds were quiet (the strength was low), and the breathing rhythm was substantially steady, as shown in the sonogram shown in Fig. 22. In Fig. 22, peaks detected between 0 second and 2 seconds, 4 seconds and 6 seconds, etc. each correspond to a single breath, a peak between 0 second and 1 second corresponds to a sound derived from an inhalation segment, and a peak between 1 second and 2 seconds corresponds to a sound derived from an exhalation segment. Thus, it was possible to extract the body sound signal of the subject from a detection signal outputted from the sensor 11. Moreover, it was found possible to determine, on the basis of the extracted body sound signal, whether the subject was in an awake state or a sleep state.

### <Body sound signal corresponding to posture>

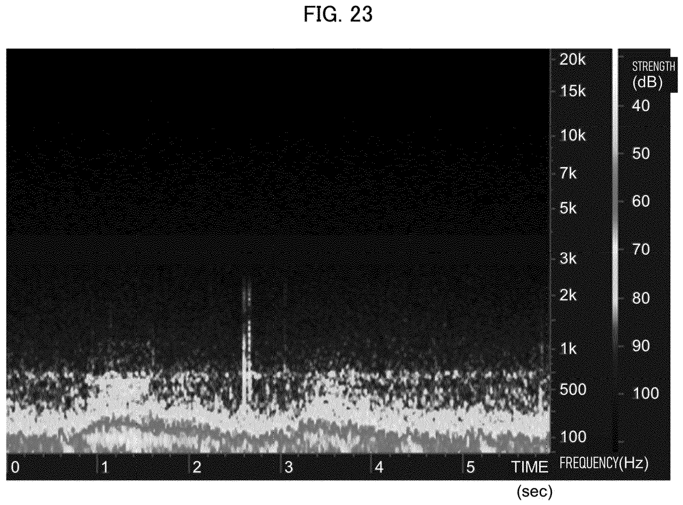

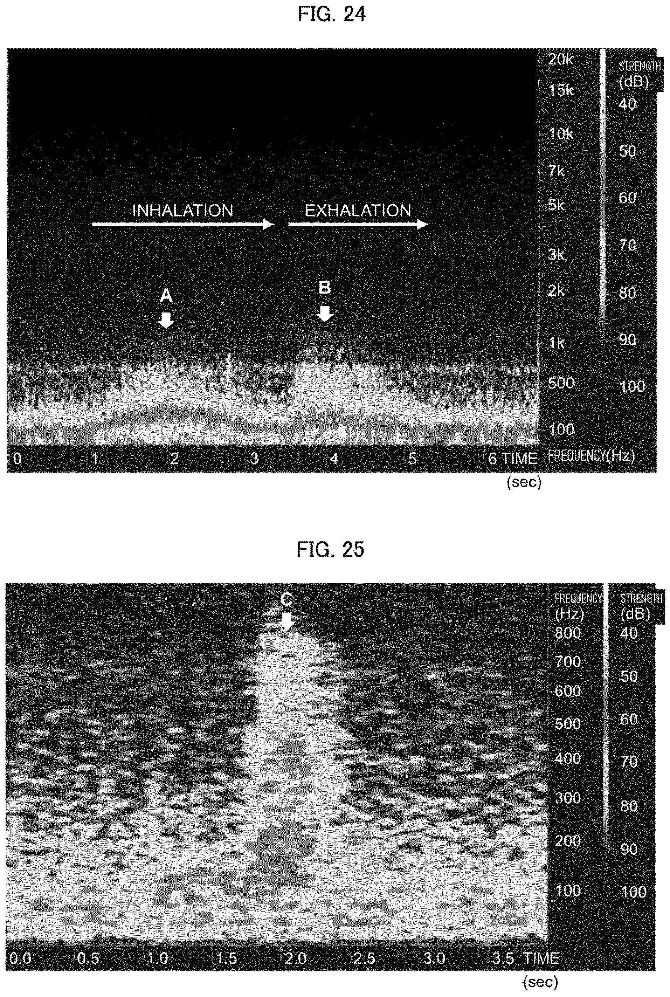

Next, in each of Figs. 23 and 24, shown is an example of a body sound signal (including a lung sound signal) that is extracted from a detection signal outputted from a sensor 11 which detects, at a position at which the sensor 11 is not in contact with a subject (healthy person), vibrations generated by the subject who changes his/her posture. Fig. 23 is a sonogram showing a body sound signal of a healthy person (left lateral decubitus position). Fig. 24 is a sonogram showing an example of a body sound signal of the healthy person (supine position). In the sonogram shown in Fig. 24, a peak seen between approximately 1 second and 3 seconds at approximately 200 Hz (A in Fig. 24) corresponds to a sound derived from an inhalation segment, and a peak seen between approximately 3 seconds and 4 seconds at approximately 200 Hz (B in Fig. 24) corresponds to a sound derived from an exhalation segment. In the sonogram shown in Fig. 23, a peak seen between approximately 1 second and 3 seconds at approximately 200 Hz corresponds to a sound derived from an inhalation segment, and a peak seen between approximately 3 seconds and 4 seconds at approximately 200 Hz corresponds to a sound derived from an exhalation segment. A comparison between the sonogram shown in Fig. 23 and the sonogram shown in Fig. 24 revealed that, in a case where the subject was in the supine position, (i) the strength of a signal corresponding to the sound derived from the inhalation segment and the strength of a signal corresponding to the sound derived from the exhalation segment were high and (ii) the peak corresponding to the sound derived from the inhalation segment and the peak corresponding to the sound derived from the exhalation segment were both clear, as compared with a case where the subject was in the left lateral decubitus position. That is, it was found possible to determine, on the basis of the extracted body sound signal, whether the posture of the subject was the left lateral decubitus position or the supine position. In addition, it was also possible to determine, on the basis of the extracted body sound signal, whether the posture of the subject was a sitting position or a decubitus position.

### <Lung sound signal including fine crackle signal or coarse crackle signal>

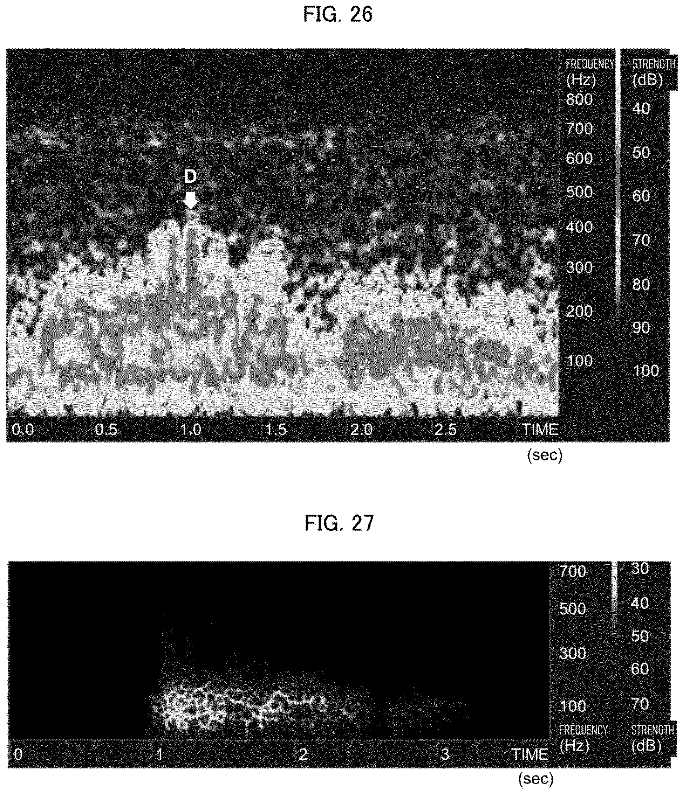

Next, detection accuracy in a case where an abnormal sound was included in a lung sound signal of a subject was verified. Fig. 25 is a sonogram showing an example of a lung sound signal of a non-healthy person which lung sound signal included a fine crackle signal. Fig. 26 is a sonogram showing an example of a lung sound signal of a non-healthy person which lung sound signal included a coarse crackle signal. In a case where fine crackles were included in lung sounds, the ratio of a component that was contained in the lung sound signal and that had a frequency of not less than 200 Hz was high. In an example shown in Fig. 25, a signal having high strength (C in Fig. 25) was detected in the wavelength range of 200 Hz to 800 Hz. In a case where coarse crackles were included in lung sounds, the ratio of a component that was contained in the lung sound signal and that had a frequency of not less than 200 Hz was high. In an example shown in Fig. 26, a signal having high strength (D in Fig. 26) was detected in the wavelength range of 200 Hz to 400 Hz.

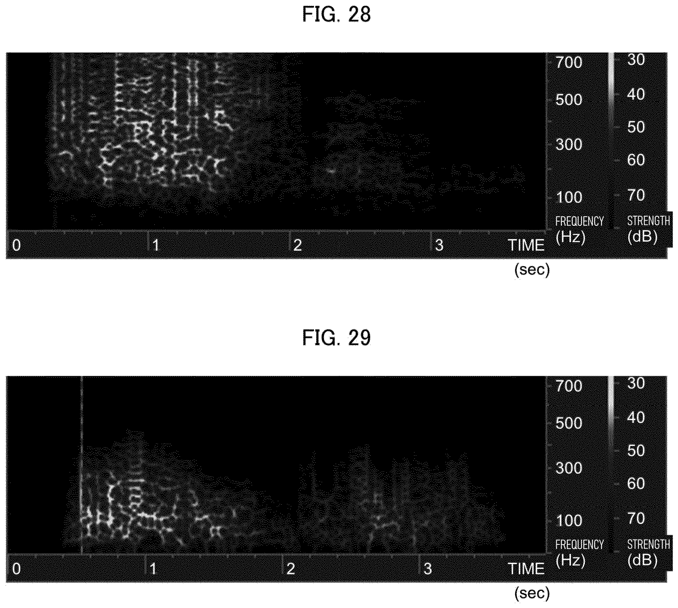

Next, the characteristics (including the presence or absence of discontinuity) of the fine crackle signal and the coarse crackle signal each included in the lung sound signal were verified with reference to Figs. 27 to 29. Fig. 27 is a sonogram obtained by extracting only a high-strength portion of a lung sound signal of a healthy person (supine position). Fig. 28 is a sonogram obtained by extracting only a high-strength portion of a lung sound signal of a non-healthy person which lung sound signal included a fine crackle signal. Fig. 29 is a sonogram obtained by extracting only a high-strength portion of a lung sound signal of a non-healthy person which lung sound signal included a coarse crackle signal. First, according to a comparison between (i) Fig. 27 and (ii) Figs. 28 and 29, in a case where a fine crackle or a coarse crackle was included in a lung sound, the ratio of a component that was contained in the lung sound signal and that had a frequency of not less than 200 Hz was obviously high. In Fig. 28, a signal having a vertical stripelike pattern was observed between 200 Hz and 700 Hz. This vertical stripe pattern results from the fact that a sound signal that was included in the lung sound signal and that had a frequency of not less than 200 Hz had discontinuity. In Fig. 29, such a vertical stripe pattern was not observed in a sound signal that had a frequency of not less than 200 Hz, but was observed in a sound signal that had a frequency of not more than 200 Hz. That is, it was found possible to determine, on the basis of the extracted lung sound signal, that an abnormal sound signal corresponded to any one of the fine crackle signal and the coarse crackle signal, in a case where the following determinations (I) and (II) were satisfied.
(I) The ratio of the component that was contained and that had a frequency of not less than 200 Hz was equal to or higher than that in a given reference body sound signal (e.g., lung sound signal of a healthy person) (YES in S604 of Fig. 14).
(II) A continuous rale was not detected in the lung sound signal (NO in S609 of Fig. 14).
Then, it was found possible to (i) determine that the abnormal sound signal was the fine crackle signal, in a case where, in the lung sound signal, the frequency of a discontinuous rale was higher than 200 Hz and (ii) determine that the abnormal sound signal was the coarse crackle signal, in a case where, in the lung sound signal, the frequency of the discontinuous rale was not more than 200 Hz (S613 in Fig. 14). It was also confirmed that it was also possible to determine, on the basis of the lung sound signal, the type of an adventitious sound signal that was included in the lung sound signal and that differed from the fine crackle signal and the coarse crackle signal.

### <Power spectrum of lung sound signal>

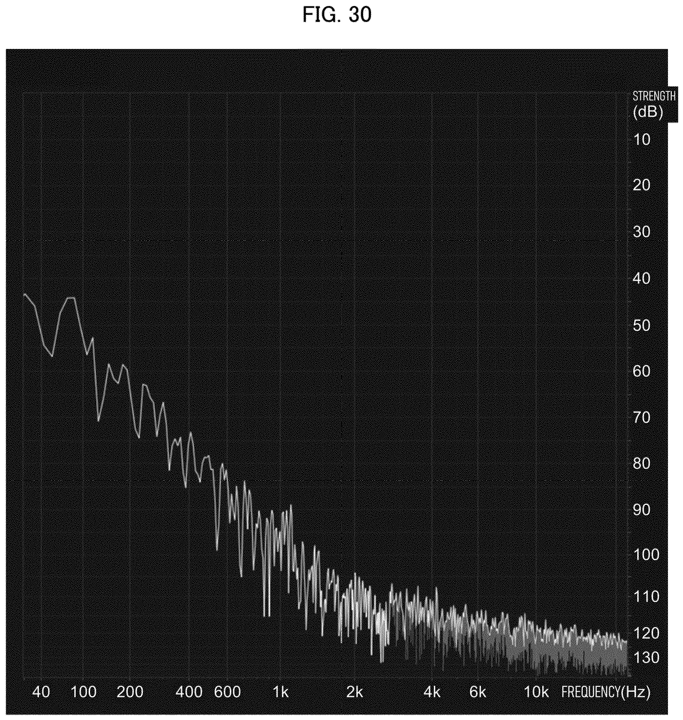

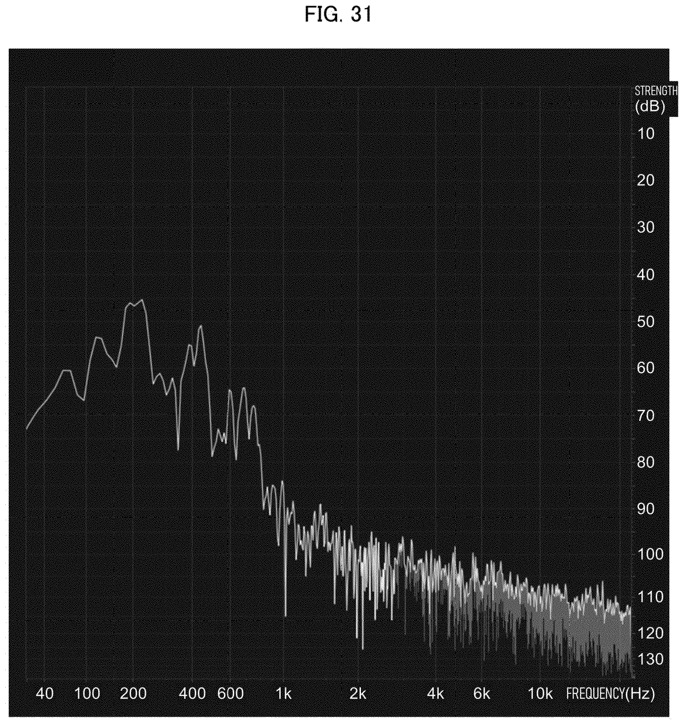

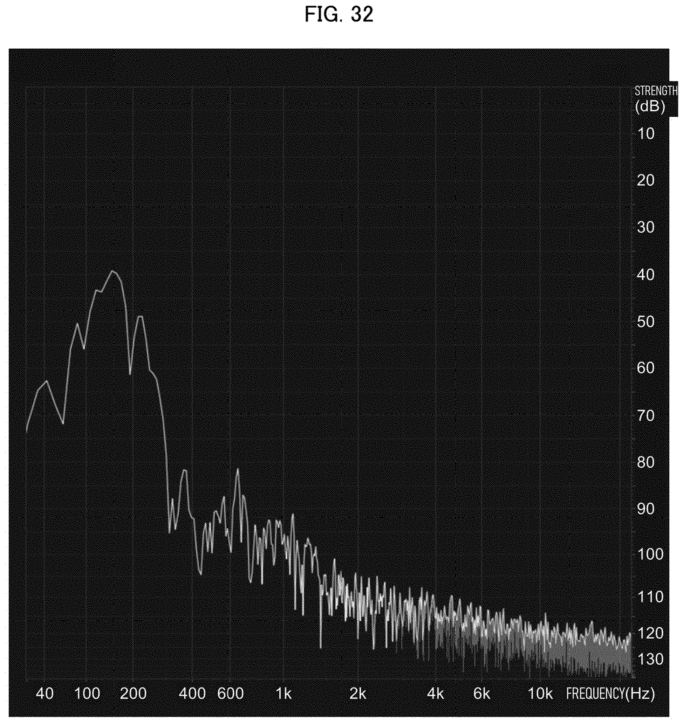

Next, power spectra of lung sound signals were examined. Fig. 30 is a view showing an example of a power spectrum of a lung sound signal of a healthy person (supine position). The lung sound signal of the healthy person showed the power spectrum in which strength became higher as a frequency became lower and the strength uniformly decreased as the frequency became higher. On the other hand, a power spectrum of a lung sound signal of each of non-healthy persons (i.e., a lung sound signal in which an adventitious sound signal was mixed) had a different shape. Fig. 31 is a view showing an example of the power spectrum of the lung sound signal of the non-healthy person which lung sound signal included a fine crackle signal. Fig. 32 is a view showing an example of the power spectrum of the lung sound signal of the non-healthy person which lung sound signal included a coarse crackle signal. In a case where the fine crackle signal was included, the power spectrum had a peak in the wavelength range of 100 Hz to 600 Hz, as shown in Fig. 31. In a case where the coarse crackle signal was included, the power spectrum had a peak in the wavelength range of 50 Hz to 400 Hz, as shown in Fig. 32. Thus, it was confirmed that mixing of the adventitious sound signal in the lung sound signal caused a change in the waveform of the power spectrum of the lung sound signal.

Figs. 31 and 32 each show the power spectrum having a clear peak that indicated mixing of an adventitious sound. However, there may be a case where a clear peak that indicates mixing of an adventitious sound is not observed in a power spectrum, depending on, for example, the degree of progress of a disease. Even in such a case, in a case where the power spectrum of the healthy person was compared with the power spectrum of each of the non-healthy persons, the power spectrum of the healthy person differed from the power spectrum of each of the non-healthy persons in ratio between a power value in a low frequency region (e.g., not more than 400 Hz) and a power value in a high frequency region (e.g., not less than 600 Hz) in the lung sound signal. That is, it was found possible to determine whether the adventitious sound signal was mixed in the lung sound signal, on the basis of the ratio between the power value of the low frequency region and the power value of the high frequency region in the lung sound signal.

### <Examples of characteristic signal>

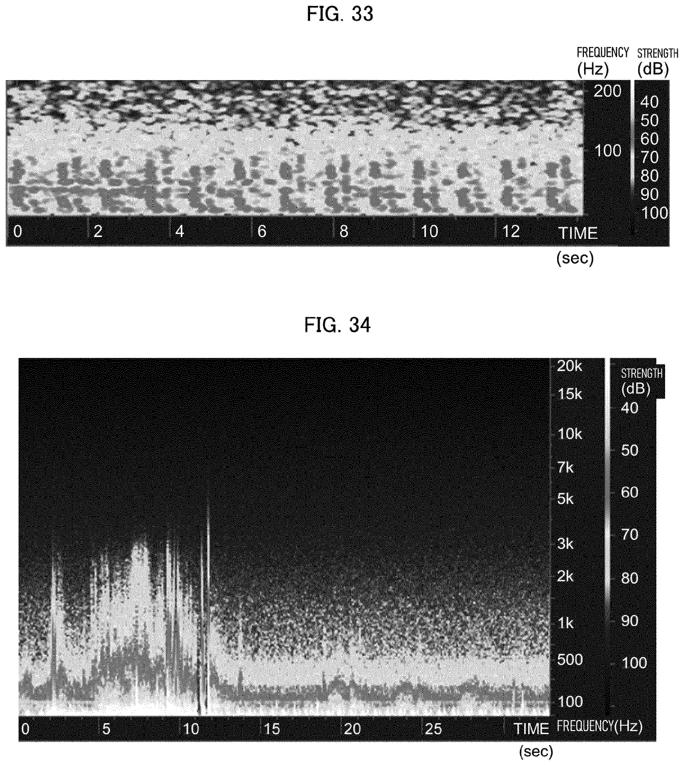

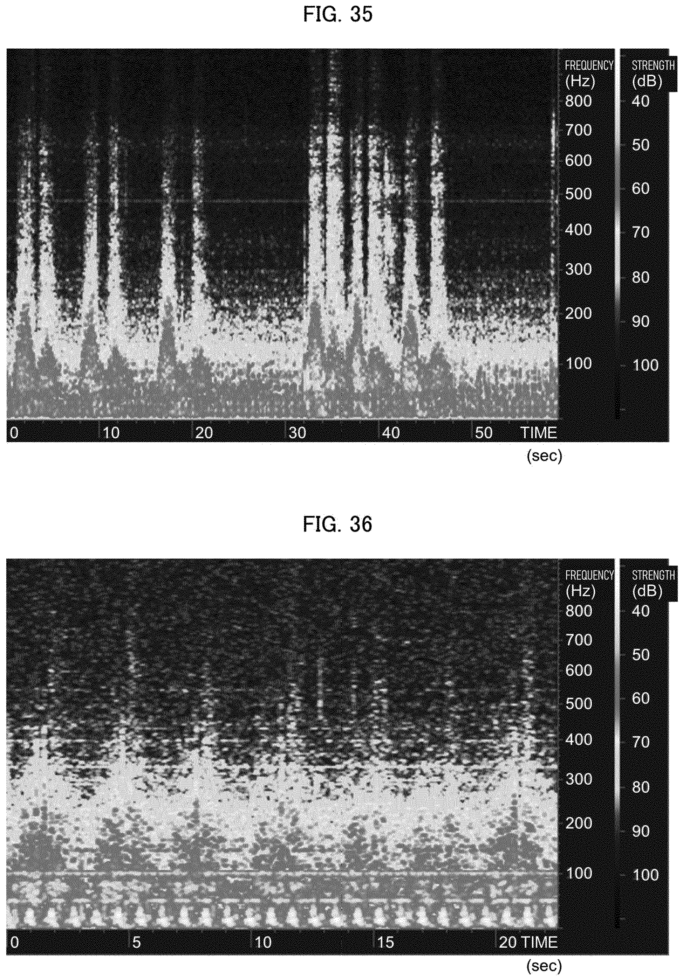

Figs. 33 to 36 each show an example of a characteristic signal included in a body sound signal. Fig. 33 is a sonogram showing an example of a heartbeat signal of a healthy person. Fig. 34 is a sonogram showing an example of a body motion signal of the healthy person. Fig. 35 is a sonogram showing an example of a breath sound signal of the healthy person (during awaking). Fig. 36 is a sonogram showing an example of a breath sound signal of the healthy person (during sleeping). It was confirmed that a characteristic signal, such as the heartbeat signal, the body motion signal, and the breath sound signal, of a subject could be extracted from a detection signal outputted from a sensor 11 that was located at a position at which the sensor 11 was not in contact with the subject.

### Reference Signs List

1, 1a, 1b, 1c Detecting device
1d Detection signal analyzing device (information processing device)
2 Server device
3 Communication device
11 Sensor
100, 100a, 100b, 100c, 100d, 100e Information processing system
101 Signal extracting section
102 Determining section (sleep determining section, posture determining section)
103 Output control section (first output control section, second output control section)
104 Site estimating section
105 Health condition determining section
106 Intervention encouragement outputting section 123 Estimation model
S1, S1a Signal extracting step
S2 Sleep determining step
S2a Posture determining step
S3 Output controlling step (first output controlling step, second output controlling step)
S6 Health condition determining step
S8 Intervention encouragement outputting step

## Claims

1. An information processing system comprising:
a signal extracting section that extracts, from a detection signal outputted from a sensor, (i) a characteristic signal which includes at least one of a heartbeat signal that indicates a heartbeat of a subject, a breathing vibration signal that indicates a breathing vibration of the subject, and a body motion signal that indicates a body motion of the subject and (ii) a body sound signal which indicates a body sound that is generated from an inside of a body of the subject, the sensor detecting, at a position at which the sensor is not in contact with the subject, vibrations generated by the subject;
a sleep determining section that determines that the subject is in a sleep state, on the basis of the characteristic signal that has been extracted; and
a first output control section that outputs the body sound signal during a time period during which it has been determined that the subject is in the sleep state, in a case where it has been determined that the subject is in the sleep state.

2. The information processing system as set forth in claim 1, wherein
the sleep determining section estimates the sleep state by inputting, into an estimation model, the characteristic signal that has been extracted by the signal extracting section, the estimation model having been trained by machine learning with use of training data in which the characteristic signal is an explanatory variable and the sleep state is a response variable.

3. An information processing system comprising:
a signal extracting section that extracts, from a detection signal outputted from a sensor, (i) a characteristic signal which includes at least one of a heartbeat signal that indicates a heartbeat of a subject, a breathing vibration signal that indicates a breathing vibration of the subject, and a body motion signal that indicates a body motion of the subject and (ii) a body sound signal which indicates a body sound that is generated from an inside of a body of the subject, the sensor detecting, at a position at which the sensor is not in contact with the subject, vibrations generated by the subject;
a posture determining section that determines that the subject is in a given posture, on the basis of at least one of the detection signal and the characteristic signal that has been extracted; and
a second output control section that outputs the body sound signal during a time period during which it has been determined that the subject is in the given posture, in a case where it has been determined that the subject is in the given posture.

4. The information processing system as set forth in claim 3, wherein
the posture determining section estimates a probability that the subject is in the given posture, by inputting, into an estimation model, at least one of the detection signal that has been detected by the sensor and the characteristic signal that has been extracted by the signal extracting section, the estimation model having been trained by machine learning with use of training data in which at least one of the detection signal and the characteristic signal is an explanatory variable and a posture of the subject is a response variable.

5. The information processing system as set forth in claim 3 or 4, wherein
the given posture is at least one of a right lateral decubitus position, a left lateral decubitus position, a supine position, a prone position, and a sitting position.

6. The information processing system as set forth in claim 1 or 3, wherein
the sensor includes a plurality of detection regions each of which outputs the detection signal, and
the signal extracting section extracts the characteristic signal and the body sound signal from each of region-specific detection signals that are outputted from the respective plurality of detection regions.

7. The information processing system as set forth in claim 6, further comprising
a site estimating section that estimates an abnormal sound occurring site in the body of the subject, on the basis of the body sound signal that has been extracted from each of the region-specific detection signals.

8. The information processing system as set forth in claim 1 or 3, wherein
the sensor obtains at least one of the body sound signal that has a frequency of not less than 100 Hz, the heartbeat signal that has a frequency of not more than 20 Hz, the breathing vibration signal, and the body motion signal.

9. The information processing system as set forth in claim 1 or 3, wherein
the sensor has a thin plate-like shape and is set on a bed on which the subject lies down.

10. The information processing system as set forth in claim 1 or 3, wherein
the body sound signal includes at least one of a lung sound signal that indicates a lung sound of the subject, a blood flow sound signal that indicates a blood flow sound of the subject, a heart sound signal that indicates a heart sound of the subject, a bowel sound signal that indicates a bowel sound of the subject, and a peristaltic sound signal that indicates a peristaltic sound of the subject.

11. The information processing system as set forth in claim 1 or 3, further comprising
a health condition determining section that determines a health condition of the subject by comparing the body sound signal and a given reference body sound signal.

12. The information processing system as set forth in claim 11, wherein
the health condition determining section
is capable of determining an inhalation segment and an exhalation segment on the basis of the breathing vibration signal that is included in the detection signal, and
determines the health condition of the subject by comparing the body sound signal and the given reference body sound signal with regard to each of the inhalation segment and the exhalation segment.

13. The information processing system as set forth in claim 11, wherein
the health condition determining section determines that the subject is in an abnormal state, in at least one of (1) a case where a lung sound signal that indicates a lung sound of the subject is attenuated or lost as compared with the given reference body sound signal, (2) a case where an abnormal sound signal that indicates an abnormal sound is mixed in the lung sound signal, and (3) a case where an adventitious sound signal that indicates an adventitious sound is mixed in the lung sound signal, and
the adventitious sound includes at least one of a coarse crackle, a fine crackle, a wheeze, a rhonchus, a stridor, a squawk, and a pleural friction rub of the subject.

14. The information processing system as set forth in claim 13, wherein
the health condition determining section
determines that the subject is affected by at least one of pneumothorax, pleural effusion, and atelectasis, in a case where the lung sound signal is attenuated or lost as compared with a lung sound signal that has been extracted from the given reference body sound signal, and
determines that the subject is affected by at least one of chronic obstructive pulmonary disease and bronchial asthma, in a case where the abnormal sound signal is mixed in the lung sound signal.

15. The information processing system as set forth in claim 13, further comprising
an intervention encouragement outputting section that outputs an intervention encouragement notification which encourages a medical intervention with respect to the subject, in a case where the health condition determining section has determined that the health condition of the subject is the abnormal state.

16. A detecting device comprising:
a sensor that detects, at a position at which the sensor is not in contact with a subject, vibrations generated by the subject;
a signal extracting section that extracts, from a detection signal outputted from the sensor, (i) a characteristic signal which includes at least one of a heartbeat signal that indicates a heartbeat of the subject, a breathing vibration signal that indicates a breathing vibration of the subject, and a body motion signal that indicates a body motion of the subject and (ii) a body sound signal which indicates a body sound that is generated from an inside of a body of the subject;
a sleep determining section that determines that the subject is in a sleep state, on the basis of the characteristic signal that has been extracted; and
a first output control section that outputs the body sound signal during a time period during which it has been determined that the subject is in the sleep state, in a case where it has been determined that the subject is in the sleep state.

17. A detecting device comprising:
a sensor that detects, at a position at which the sensor is not in contact with a subject, vibrations generated by the subject;
a signal extracting section that extracts, from a detection signal outputted from the sensor, (i) a characteristic signal which includes at least one of a heartbeat signal that indicates a heartbeat of the subject, a breathing vibration signal that indicates a breathing vibration of the subject, and a body motion signal that indicates a body motion of the subject and (ii) a body sound signal which indicates a body sound that is generated from an inside of a body of the subject;
a posture determining section that determines that the subject is in a given posture, on the basis of at least one of the detection signal and the characteristic signal that has been extracted; and
a second output control section that outputs the body sound signal during a time period during which it has been determined that the subject is in the given posture, in a case where it has been determined that the subject is in the given posture.

18. The detecting device as set forth in claim 16 or 17, further comprising
a health condition determining section that determines a health condition of the subject by comparing the body sound signal and a given reference body sound signal.

19. The detecting device as set forth in claim 18, further comprising
an intervention encouragement outputting section that outputs an intervention encouragement notification which encourages a medical intervention with respect to the subject, in a case where the health condition determining section has determined that the health condition of the subject is an abnormal state.

20. A server device comprising:
a health condition determining section that obtains the body sound signal which has been outputted from the detecting device recited in claim 16 or 17 and that determines a health condition of the subject by comparing the body sound signal and a given reference body sound signal; and
an intervention encouragement outputting section that outputs an intervention encouragement notification which encourages a medical intervention with respect to the subject, in a case where the health condition determining section has determined that the health condition of the subject is an abnormal state.

21. A server device comprising
an intervention encouragement outputting section that obtains a determination result which has been outputted from the detecting device recited in claim 18 and which relates to the health condition and that outputs an intervention encouragement notification which encourages a medical intervention with respect to the subject, in a case where the determination result that has been obtained indicates that the health condition of the subject is an abnormal state.

22. An information processing device comprising:
a signal extracting section that obtains a detection signal outputted from a sensor which detects, at a position at which the sensor is not in contact with a subject, vibrations generated by the subject and that extracts, from the detection signal, (i) a characteristic signal which includes at least one of a heartbeat signal that indicates a heartbeat of the subject, a breathing vibration signal that indicates a breathing vibration of the subject, and a body motion signal that indicates a body motion of the subject and (ii) a body sound signal which indicates a body sound that is generated from an inside of a body of the subject;
a sleep determining section that determines that the subject is in a sleep state, on the basis of the characteristic signal that has been extracted; and
a first output control section that outputs the body sound signal during a time period during which it has been determined that the subject is in the sleep state, in a case where it has been determined that the subject is in the sleep state.

23. An information processing device comprising:
a signal extracting section that obtains a detection signal outputted from a sensor which detects, at a position at which the sensor is not in contact with a subject, vibrations generated by the subject and that extracts, from the detection signal, (i) a characteristic signal which includes at least one of a heartbeat signal that indicates a heartbeat of the subject, a breathing vibration signal that indicates a breathing vibration of the subject, and a body motion signal that indicates a body motion of the subject and (ii) a body sound signal which indicates a body sound that is generated from an inside of a body of the subject;
a posture determining section that determines that the subject is in a given posture, on the basis of at least one of the detection signal and the characteristic signal that has been extracted; and
a second output control section that outputs the body sound signal during a time period during which it has been determined that the subject is in the given posture, in a case where it has been determined that the subject is in the given posture.

24. A control method which is carried out by one or more information processing devices, comprising:
a signal extracting step of extracting, from a detection signal outputted from a sensor, (i) a characteristic signal which includes at least one of a heartbeat signal that indicates a heartbeat of a subject, a breathing vibration signal that indicates a breathing vibration of the subject, and a body motion signal that indicates a body motion of the subject and (ii) a body sound signal which indicates a body sound that is generated from an inside of a body of the subject, the sensor detecting, at a position at which the sensor is not in contact with the subject, vibrations generated by the subject;
a sleep determining step of determining that the subject is in a sleep state, on the basis of the characteristic signal that has been extracted; and
a first output controlling step of outputting the body sound signal during a time period during which it has been determined that the subject is in the sleep state, in a case where it has been determined that the subject is in the sleep state.

25. A control method which is carried out by one or more information processing devices, comprising:
a signal extracting step of extracting, from a detection signal outputted from a sensor, (i) a characteristic signal which includes at least one of a heartbeat signal that indicates a heartbeat of a subject, a breathing vibration signal that indicates a breathing vibration of the subject, and a body motion signal that indicates a body motion of the subject and (ii) a body sound signal which indicates a body sound that is generated from an inside of a body of the subject, the sensor detecting, at a position at which the sensor is not in contact with the subject, vibrations generated by the subject;
a posture determining step of determining that the subject is in a given posture, on the basis of at least one of the detection signal and the characteristic signal that has been extracted; and
a second output controlling step of outputting the body sound signal during a time period during which it has been determined that the subject is in the given posture, in a case where it has been determined that the subject is in the given posture.

26. A control method which is carried out by one or more server devices, comprising:
a health condition determining step of obtaining the body sound signal which has been outputted from the one or more information processing devices each of which carries out the control method recited in claim 24 or 25 and determining a health condition of the subject by comparing the body sound signal and a given reference body sound signal; and
an intervention encouragement outputting step of outputting an intervention encouragement notification which encourages a medical intervention with respect to the subject, in a case where it has been determined, in the health condition determining step, that the health condition of the subject is an abnormal state.

27. A program for causing a computer to function as the information processing system recited in claim 1, the program causing the computer to function as the signal extracting section, the sleep determining section, and the first output control section.

28. A program for causing a computer to function as the information processing system recited in claim 3, the program causing the computer to function as the signal extracting section, the posture determining section, and the second output control section.

29. A program for causing a computer to function as the server device recited in claim 20, the program causing the computer to function as the health condition determining section and the intervention encouragement outputting section.

30. A program for causing a computer to function as the server device recited in claim 21, the program causing the computer to function as the intervention encouragement outputting section.
